# EUROPEAN PATENT APPLICATION

(11) **EP 2 236 144 A1**
(43) Date of publication of application: **06.10.2010**
(21) Application number: 09700138.2
(22) Date of filing: 05.01.2009
(51) Int. Cl.: A61K 33/00, A61K 33/06, A61K 33/24, A61K 33/38, A61K 35/02, A61P 17/18, A61P 39/06

(54) **EXTERNAL PREPARATION FOR PREVENTIVE OR THERAPEUTIC USE**

(30) Priority: 28.12.2007 JP 2007340283; 19.05.2008 JP 2008130957
(71) Applicant: MIZ Co., Ltd., Fujisawa-shi Kanagawa 251-0871 (JP)
(72) Inventor: SATOH, Bunpei, Fujisawa-shi Kanagawa 251-0871 (JP); SATOH, Youhei, Fujisawa-shi Kanagawa 251-0871 (JP); SATOH, Fumitake, Fujisawa-shi Kanagawa 251-0871 (JP)
(74) Representative: TER MEER - STEINMEISTER & PARTNER GbR
(86) International application number: PCT/JP2009/050010
(87) International publication number: WO 2009/084743

(57) **Abstract**

An external medicine includes an active hydrogen generating agent, which generates active hydrogen by bringing multiple agents into contact, and/ or an active hydrogen generating agent, which activates generated hydrogen molecules.
Representative drawing:... None

## Description

### Technical Field

The present invention relates to an external medicine for treatment or prevention.

### Background Art

Oxidant stress due to active oxygen or free radicals contributes to many diseases; however, a method of using antioxidative activity of active hydrogen as a method to solve this problem is well known (Patent Document 1). An antioxidation method of changing an object of antioxidation, which is either in an oxidation state due to a deficiency of electrons or is to be protected from oxidation, to be in a reduced state with sufficient electrons through a process of breaking down into the active hydrogen using a catalyst acting on hydrogen molecules or substrates, is proposed in the document.

On the other hand, a method of generating hydrogen molecules by bringing water or acid into contact with metal magnesium is well known (Patent Documents 2 and 3).

However, with the technology disclosed in Patent Document 1, there are problems of low solubility of hydrogen molecules into a water medium and easiness of dispersion and loss from the medium, and also a problem that a desired reducing power upon practical application is not exhibited, such as the hydrogen molecules are wasted when the hydrogen molecules and the medium exist within the same system and an oxide other than the object for oxidation such as oxygen exists therein.

Moreover, a strong result as with the present invention described later cannot be achieved by a simple combination of the generation method for hydrogen molecules using metal magnesium disclosed in Patent Document 2 or 3, and the hydrogen-dissolved water including a catalyst disclosed in Patent Document 1.

[Patent Document 1] WO 2003/002466

[Patent Document 2] Japanese Unexamined Patent Application Publication No. 2006-199866
[Patent Document 3] Japanese Unexamined Patent Application Publication No. 2003-010865

### DISCLOSURE OF THE INVENTION

### [Objective of the Invention]

The objective of the present invention is to provide an external medicine not limited by solubility in a medium (solvent) for hydrogen molecules when using reducing power of active hydrogen as an external medicine for treatment or prevention.

### [Means of Solving the Problem]

The present invention resolves the aforementioned problem by directly providing active hydrogen, which is obtained through generation of active hydrogen or activation of hydrogen molecules, to an oxidized region due to a deficiency of electrons or region to be protected from oxidation so as to bring the region into a reduced state with sufficient electrons.

### [Result of the Invention]

According to the present invention, reducing power of active hydrogen may be used as an external medicine for treatment or prevention.

### BRIEF DESCRIPTION OF DRAWINGS

FIG. 1 is a graph showing test results of Working Examples 3 to 9;
FIG. 2 is a graph showing test results of Working Example 10;
FIG. 3 is a graph showing results (whole area) of a dermatitis inhibition test;
FIG. 4 is a graph showing results (back) of the dermatitis inhibition test;
FIG. 5 is a graph showing results (auricles) of the dermatitis inhibition test;
FIG. 6 is a graph showing results of comparative reference examples of the dermatitis inhibition test;
FIG. 7 is a control ESR spectrum for hydroxy radical elimination measurement;
FIG. 8 is a Mg supernatant liquid ESR spectrum for hydroxy radical elimination measurement;
FIG. 9 is a Mg ESR spectrum for hydroxy radical elimination measurement; and
FIG. 10 shows a Mg ESR spectrum and DMPO-H and DMPO-OH spectrums for hydroxy radical elimination measurement.

### BEST MODE FOR CARRYING OUT THE INVENTION

According to an embodiment of the present invention, an external medicine for treatment or prevention containing either an agent for generating active hydrogen or an agent for generating hydrogen molecules and activating them using a catalyst (hereafter, both are referred to as active hydrogen generating agent) may be used for an object of antioxidation which is a target region (for example, either an object of antioxidation which is oxidized due to a deficiency of electrons in skin or a mucous membrane of a person or animal, or an object to be protected from oxidation). Such an external medicine may be constituted by multiple agents. For example, active hydrogen may be applied to an object of antioxidation or target region by including an electron donor or a hydride ion donor in a first agent, including a substance containing a proton donor in a second agent, and bringing the first agent into contact with the second agent . Moreover, inclusion of a catalyst in one of the aforementioned multiple agents allows activation again of the hydrogen molecules that generate due to reaction between the active hydrogen and protons or reaction between active hydrogen molecules (the target region is exposed to the active hydrogen as a result.)

'Active hydrogen generating agent' in this specification is a concept indicating an agent which can generate active hydrogen molecules through a chemical, physical, electrical, or mechanical means, an agent which generates hydrogen molecules and accelerates a reaction making active hydrogen using a catalyst, or an agent which generates hydrogen molecules and accelerates a reaction making active hydrogen through the hydrogen molecules being oxidized into active oxygen or strong oxidative radical. It also includes, for example, a chemical compound (hydrogen compound) containing a hydrogen element such as a hydrocarbon compound or organic hydride as a component, a substance characteristic of storing and releasing hydrogen molecules such as a hydrogen storing alloy or liposome, and a substance which itself does not contain hydrogen but can react with the hydrogen compound or hydrogen storing alloy to generate hydrogen.

'Electron donor' in this specification indicates a substance which releases an electron and transfers the electron to a proton. Electron donors include those characteristic of directly transferring an electron to a proton, those characteristic of first releasing an electron to a solvent and then transferring it to a proton, and those characteristic of first transferring an electron to a catalyst or electron carrier, which smoothly transfers the electron to a proton, and then transferring it to a proton.

Examples of electron donors include 'metals' including alkali metals and alkaline-earth metals, preferably metals with a higher tendency of ionization than hydrogen such as lithium, rubidium, potassium, barium, strontium, calcium, sodium, magnesium, aluminum, manganese, zinc, chromium, iron, cadmium, cobalt, nickel, tin, lead, alloy compound or complex compound of these metals, or composite thereof or the like. However, use of other metals, alloy compounds or complex compounds of these other metals, or a composite thereof is not to be prohibited. In view of safety in handling, safety to living organisms, and the like, metal magnesium, magnesium hydride, zinc and similar metals can be called preferred metals.

'Hydride ion donor' in this specification indicates a substance which releases a hydride ion and transfers the hydride ion to a proton. Hydride ion donors include those characteristic of directly transferring a hydride ion to a proton, those characteristic of first releasing a hydride ion to a solvent and then transferring it to a proton, and those characteristic of first transferring a hydride ion to a catalyst or electron carrier, which smoothly transfers the hydride ion to a proton, and then transferring it to a proton.

Examples of hydride ion donors include metal hydrides such as calcium hydride, magnesium hydride or similar hydrides obtained by applying hydrogen gas to metal, hydrides of groups 13 and 14 elements with metallic quality such as sodium borohydride and the like.

'Proton donor' in this specification is a concept including substances which donate a proton in a solvent, substances which are ionized by donating and receiving a proton during self separation even though an isolated proton is not donated, and compounds having a hydrogen element as a component, namely hydrogen compounds.

Note that while hydrogen derived from the hydroxyl group (-OH) may be reduced when an amphoteric metal such as zinc, aluminum, lead, or tin is used as an electron donor, such a hydroxyl compound may also be included in the concept of the proton donor in this specification. Examples of the proton donor, although not limited to these, include water, formic acid, methanol, ethanol, 1-propanol, 2-propanol, 1-butanol, and acetic acid, which are amphoteric solvents or protic solvents.

Hydrogen molecules are generated by a substance containing an electron donor or a hydride ion donor making contact with a substance containing a proton donor. While not limited thereto, this process is basically described by the reaction formulas given below.

### <Formula 1>

2H(+) + 2e(-) → H₂ ... (a)

H(+) + H(-) → H₂ ... (b)

Namely, according to reaction formula (a), electrons (e(-)) donated from an agent containing an electron donor generate a hydrogen molecule (H₂) through reduction of a proton (H(+) donated from an agent containing a proton donor.

Similarly, according to reaction formula (b), a hydride ion (H(-)) donated from an agent containing a hydride ion generates a hydrogen molecule (H₂) through reduction of a proton (H(+) donated from an agent containing a proton donor. Note that as described later, e(-) and H(-) in formulas (a) and (b) are concepts included in the 'active hydrogen' of this specification.

Reduction of a proton here is a concept including reduction of a free proton donated from a proton donor, an attached proton, or a proton donor itself. Also, it is a concept including reduction of a compound having a hydrogen element as a component, namely a hydrogen compound by a reducer of hydrogen in the hydrogen compound.

'Acid' in this specification includes chemical species such as citric acid, acetic acid, succinic acid, gluconic acid, lactic acid, malic acid, phosphoric acid, hydrochloric acid, sulfuric acid, and the like, or acid (solution) created through an electrochemical means such as electrolysis, but the present invention is not limited to these examples.

Moreover, in the case of using the aforementioned amphoteric metal as an electron donor, use of a 'basic substance' such as sodium hydroxide is possible.

'Catalyst' in this specification includes all of those that have a function of accelerating a reaction of breaking down generated hydrogen molecules into active hydrogen as a product. Examples thereof are precious metal microparticles, metal complexes, hydrogen oxidation-reduction hydrogenase enzymes, active site model compounds thereof, ultrasonic waves, and electromagnetic waves.

Note that while not intending to limit the present invention, precious metal particle in this specification is a concept including platinum, palladium, rhodium, iridium, ruthenium, gold, silver, and rhenium, salts of these precious metal elements, alloy compounds, complex compounds, precious metal particles themselves, and mixtures thereof.

Moreover, precious metal microparticle (catalyst) referred to in this specification assumes a particle with a diameter of 1 nm to 0.5 µm, which is said to generally present fundamental behavior as a colloid. However, a particle diameter that increases catalytic activity of Pt colloid, for example, when Pt colloid is employed as a precious metal microparticle, should be in the range of 1 to 10 nm, more preferably 4 to 6 nm. This is a particle diameter derived from a trade-off relationship between bringing out the inherent property as a precious metal, and increasing surface for improvement in catalytic property.

Note that while not limited thereto, the process by which generated hydrogen molecules are activated is basically described by the reaction formula given below.

### <Formula 2>

H2 → 2H• ... (c)

2H• → 2H(+) + 2e(-) ... (d)

or

H2→ H(+) + H(-) ... (e)

H(-) → H(+) + 2e(-) ... (f)

The concept of active hydrogen includes e(-) in formula (a) and H(-) in formula (b), as well as both atomic hydrogen (H•) as shown in reaction formula (c) and hydride ion (H(-)) as shown in reaction formula (e), and all types of theoretically possible aspects of activated hydrogen molecules, such as a hydrogen molecule ion or protonated hydrogen molecule. As shown in reaction formulas (d) and (f), activation of a single hydrogen molecule provides two electrons.

At the same time, merely a hydrogen molecule instead of active hydrogen also shows reduction action on a radical with high reactivity such as a hydroxyl radical. While this results from the hydroxyl radical being reduced through drawing out of hydrogen by the hydroxyl radical to oxidize the hydrogen molecules, 'active hydrogen' in this specification also includes hydrogen molecules forcibly activated by such a highly reactive radical.

In the case of configuring an external medicine for treatment or prevention that selectively acts on such a highly reactive radical, the catalyst for activating hydrogen molecules does not always need to be included in the component. However, even in that case, use of an active hydrogen generating agent capable of generating 'hydrogen in a nascent state' such as e(-) in formula (a) and H(-) in formula (b) in the process of hydrogen generation is preferred. Moreover, even if a catalyst is used or not, there is still the merit of this external medicine of being able to generate a large quantity of active hydrogen at an object of antioxidation without including hydrogen molecules beforehand.

'Object of antioxidation' in this specification includes objects of antioxidation in an oxidized state due to deficiency of electrons in an organism or all objects of antioxidation to be protected from oxidation.

While not intending to limit the present invention, examples of objects of antioxidation involved in human diseases are hepatic and renal damage due to chemicals and harmful substances, ischemic reperfusion disorder, circulatory system diseases such as arteriosclerosis, alimentary disorders such as gastric ulcer and gastric mucosal disorder, respiratory diseases, arteriosclerosis, complications with diabetes (e.g., high blood pressure, stroke, heart attack), cataract, skin diseases, various inflammatory diseases, neurological disorders, cancer, aging, menopause, erectile dysfunction (ED), depressive psychosis, gum disease, osteoporosis, autoimmune diseases such as rheumatism, stiff neck, sensitivity to cold, high blood pressure, and senile dementia.

More specifically, examples of the skin area include but are not limited to various cutaneous manifestations such as acne, pigmented spots, and wrinkles deriving from oxidation, aging or photoaging of skin and/ or mucous membranes without distinction of epidermis or dermis, or pigmentation such as liver spots, freckles, sunlight moles, and dullness, which are esthetic and cosmetic subjects, and subjects of skin diseases to which oxidant stress contributes directly and indirectly, such as external injury, eczema, hives, erythema, pupuric skin angitis, general angitis, keratonosis, hydroa, pustulosis, metabolic disorders, dysplasia, granulomatous disease, collagen disease, infectious diseases, nevus, phacomatosis, benign tumors and pigmentation disorders, malignant tumors, disorders of the sebaceous gland and the sudoriferous gland, hair diseases, nail diseases, solar keratosis, xeroderma pigmentosum, burn injuries, bed sores, reddening, crusta, xerosis, edama, abrasion, itchiness, and soreness.

Moreover, 'external medicine' in this specification includes agents injected in or applied on to a body surface such as skin or a mucous membrane, as well as agent injected in or applied on to bodily regions normally not externally exposed, such as internal organs, blood vessels, and nerve tissue, which are exposed by cutting open the body during an operation or the like, for example, and concepts thereof. Similarly, an organ preservative used at the time of organ preservation or organ transplant and concept thereof are also included.

Furthermore, the present invention does not inhibit combination of an existing percutanaeous absorption promoter for helping action in portions deeper than skin, or other compositions of arbitrary components generally used as external medicines.

Even in such arbitrary components, a dispersant is especially important when this external medicine for treatment or prevention adopts a form such that the metal or metal hydride is retained in a solvent. This is because in order to disperse evenly the metal or metal hydride without being precipitated in the solvent (dispersion medium), use of a dispersant while diminishing the particle diameter of the metal is desired.

There are many such dispersants that may be used such as a cellulose derivative such as hydroxypropylcellulose and methylcellulose, as well as glycerin (which also functions as a moisturizing agent), sodium alginate, and sodium polyacrylate. Use of agar, collagen, gelatin, and the like is also possible.

The sequence of dispersing the metal or metal hydride in a solvent (dispersion medium) is to first place the metal or metal hydride in the solvent (dispersion medium), and leave it to stand for several hours to approximately one day. In doing so, the dispersant is added once a passive film is formed on the surface of the metal or metal hydride. At this time, the smaller the particle diameter of the metal or metal hydride and the larger the surface area, the more time required for formation of the passive film.

Note that when forming the passive film, film formation may be aggressively prompted appropriately using at the same time a corrosion inhibitor such as a phosphate (e.g., sodium polyphosphate), silicate, amine, polymer, salt of oxo acid (e.g., molybdate, nitrous acid), and the like.

Alternatively, in addition to or instead of formation of a passive film, oil coating, microencapsulation or the like may be performed to further block contact to the solvent (dispersion medium).

Addition of a metal or metal hydride to the solvent (dispersion medium) to which the dispersant is added before a passive film is formed on the metal or metal hydride is unfavorable. This is because when a protic solvent such as water is used as the solvent (dispersion medium), the metal or metal hydride releases hydrogen while gradually reacting with the protic solvent, and thus when the solvent has viscosity, hydrogen gas bubbles become foamy, thereby inhibiting uniform dispersion of metal particles.

Moreover, the dispersant has roles of dispersing the aforementioned metal particles uniformly, increasing retaining ability in an object of antioxidation, and keeping hydrogen and active hydrogen generated in the antioxidation subject from being released in the air.

Furthermore, when a substance containing a metal or metal hydride adopts a form such that the metal is retained in the solvent (dispersion medium), use of a nonpolar solvent such as acetone or an aprotic solvent such as dimethyl sulfoxide (DMSO) may be considered in order to prevent loss of amount of active ingredients due to reaction with the protic solvent. Alternatively, combining a nonpolar solvent and an aprotic solvent in an appropriate ratio in the protic solvent may be considered. The combination ratio of the nonpolar solvent and/ or the aprotic solvent in the protic solvent should be 1 wt% or greater, preferably 20 wt% or greater, more preferably 30 wt% or greater, most preferably 50 wt% or greater. Alternatively, from a chemical reaction formula between a metal or metal hydride contained in a solvent (dispersion medium) and a protic solvent used as the solvent (dispersion medium), quantity of the protic solvent required for all of the metal or metal hydride contained in the solvent to react may be calculated so as to adjust quantity of the combination of the nonpolar solvent and the aprotic solvent such that it is less than quantity of the protic solvent in the solvent (dispersion medium).

Such nonpolar solvent includes but is not limited to the aforementioned acetone, hexane, benzene, toluene, diethyl ether, chloroform, ethyl acetate, and dichloromethane. Such aprotic solvent includes but is not limited to the aforementioned DMSO, tetrahydrofuran, acetonitrile, and N,N-dimethylformamide. More specifically, retaining a metal with significantly high reactivity such as calcium hydride in a nonpolar solvent, an aprotic solvent, or a low-protogenic protic solvent is desired.

Alternatively, another method for preventing reaction between the metal or metal hydride and the protic solvent within the solvent (dispersion medium) should combine a nonpolar solvent and an aprotic solvent in the solvent (dispersion medium), and based on the law of chemical equilibrium, combine a hydroxide, such as sodium hydrate, which ionizes in the protic solvent and emits hydroxide ions, thereby controlling beforehand a reaction (forward reaction) generating hydrogen molecules while generating hydroxide (hydroxyl ions). Liquidity of the solvent (dispersion medium) at that time should have a desired alkalinity of pH 7 to 14, preferably 7.5 to 13.5, further preferably 8 to 13, most preferably 8.5 to 12.5. Adoption of a structure such that by adding a second agent described later having an acidic liquidity to such solvent (dispersion medium), the aforementioned reaction (forward reaction) starts again (in a target region of skin or a mucous membrane).

Moreover, a well-known chelating agent or ion-exchange sequestering agent may be appropriately combined so as to make consideration for anxiety of metal allergy regarding metal ions liquating out from the metal or metal hydride.

According to the external medicine for treatment or prevention of this specification, since it is unnecessary to include hydrogen molecules in a solvent (medium) beforehand when the reducing power and antioxidation power of active hydrogen are utilized, there is no need to care about the poor solubility of the hydrogen molecules into the solvent, or temporal dispersion and loss thereof into the air. Moreover, since the generated active hydrogen or the hydrogen molecules activated via a catalyst makes an object of antioxidation, which is oxidized in an organism due to a deficiency of electrons or object of antioxidation in an organism to be protected from oxidation, such as an object of antioxidation or target region such as a wrinkle, pigmented spot, or dark spot of skin or a mucous membrane, for example, be in a reduced state with sufficient electrons, there is no fear of the active hydrogen being wasted for dissolved oxide before reaching the object of antioxidation or target as in the case where the hydrogen molecules and the catalyst coexist within the system from the beginning.

In this manner, prominent characteristics of the external medicine for treatment or prevention according to the present invention are that the hydrogen molecules or active hydrogen is not included in the solvent beforehand but is generated for the first time at the time of administration of the medicine, and that generation of active hydrogen or activation of hydrogen via a catalyst basically occurs in the object of antioxidation or the target and thus not much of the active hydrogen is wasted by an oxidizing agent other than the target, and therefore most of it is used for the object of antioxidation or target region.

Moreover, by including an existing component in at least one substance of the many agents constituting the external medicine for treatment or prevention of the present invention, a synergistic effect of that component and hydrogen or active hydrogen may be expected. Examples of such component, which do not limit the present invention, include those used in treatment of the aforementioned diseases.

A representative embodiment is described next.

A liquid containing metal magnesium impalpable powder as a first agent and citric acid solution or citric acid solution containing platinum microparticles as a second agent are poured into separate containers and stored until time of use. Alternatively, even if it is a single container, a dual dispenser type container, which has a structure having spaces for accommodating two agents separately within the container, where the two agents are released separately at the same time by pressing a nozzle at the top of the container to bring the two agents into contact with each other for the first time, may be used.

When using this external medicine for treatment or prevention on the object of antioxidation or target region of skin or a mucous membrane, apply the first agent and the second agent in an appropriate ratio on the object of antioxidation and let it sit for a few seconds to several tens of minutes. At this time, it is preferable for the first agent and the second agent to be given an appropriate amount of viscosity using a dispersant or the like in view of retentivity of the external medicine for treatment or prevention on the object of antioxidation. The dispersant may also function as a protective film for the generated active hydrogen or hydrogen molecules.

The first agent and the second agent mutually mixed and applied on the object of antioxidation begin to release active hydrogen and hydrogen molecules. The mechanism thereof is assumed to remove the passive film covering the metal magnesium microparticles within the first agent using acid in the second agent, release active hydrogen and hydrogen molecules due to reaction of water or hydrated protons with the exposed active surface of metal magnesium, and generate magnesium hydrate. Namely, representing this by a reaction formula gives

### <Formula 3>

Mg + 2H₂O → Mg(OH)₂ + H₂

and elementary reactions

### <Formula 4>

Mg → Mg(2+) + 2e(-)

and

### <Formula 5>

2H₂O + 2e(-) → 2OH(-) + H₂ (case of reaction with water)

2H(+) + 2e(-) → H2 (case of reaction with hydrated protons)

can be considered as reaction mechanisms thereof. However, this assumption is not to limit the present invention.

Note that when a metal hydride such as calcium hydride is used instead of metal magnesium here, the formula becomes

### <Formula 6>

CaH₂ + H₂0→ CaO + 2H₂, and reaction thereof is basically represented by the reaction formula,

### <Formula 7>

H(+) + H(-)→H₂

where H(-) is a substance called a hydride ion. Diameter of the hydride ion has 3 angstroms, which is approximately three times larger than that of H. A reason thereof is thought to be that since two electrons coexist along the 1s orbit, an electron cloud spreads due to repulsion between the electrons. An extensive electron cloud means that an electron is easily removed.

Incidentally, as mentioned above, acid takes on a role of supplying a hydrated proton as well as role of removing the passive film from the metal magnesium. Generally, while the passive film may be viewed as a composite of oxide and hydroxide, acid is assumed to have a mechanism of preventing formation of such passive film made of oxide and hydroxide, as well as dissolving a formed passive film. Note that it is desirable for a metal surface exposing action of the external medicine for treatment or prevention to be appropriately reinforced, not exclusively by acid.

In the case of using water of the neutral region instead of acid as the second agent, generation rate of hydrogen molecules is generally gradual. In such case, positive use of mechanical stimulus such as digital compression to expose the active surface of the metal magnesium and making the particle diameter of the metal magnesium as small as possible to increase surface area contributing to the reaction can be recommended.

As described above, the active hydrogen or hydrogen molecules activated via a catalyst make the object of antioxidation or target region of skin or a mucous membrane, in which the active hydrogen is generated or the hydrogen molecules are activated via the catalyst, change to be in a reduced state with sufficient electrons. In this series of processes, since generation of active hydrogen or activation of hydrogen molecules and consumption of the activated hydrogen (active hydrogen) occur almost simultaneously on the object of antioxidation or target, reducing power of the active hydrogen, which could not be effectively utilized for an organism since it cannot exist stably, may be actually utilized.

Even if the generated active hydrogen or activated hydrogen molecules are left as is, the object of antioxidation or target region of skin or a mucous membrane is automatically changed to be in a reduced state with sufficient electrons; however, in this case, in order to make the reaction arise more quickly and more effectively, it is preferable to apply the first agent and the second agent to the object of antioxidation or target region so as to impregnate them. Alternatively, when the external medicine for treatment or prevention is given viscosity, the first agent and the second agent may be mixed thoroughly beforehand and the resulting creamy mixture applied to the object of antioxidation. Note that since mechanical stimulus by digital compression has an effect of removing the metal magnesium passive film, the active surface of the metal magnesium may be re-exposed if mechanical stimulation by digital compression or the like is applied again at the timing when release of hydrogen gas bubbles begins to reduce.

Regarding particle diameter of impalpable powder of metal or metal hydride or precious metal microparticles, regardless of the description of the aforementioned preferred particle diameter of precious metal microparticles, the impalpable powder or precious metal microparticles used for the external medicine for treatment or prevention may be adjusted to have a size large enough so as not to permeate through the skin, namely a particle diameter for pores or exocrine glands, such as sweat glands and sebaceous glands not to allow the impalpable powder or precious metal microparticles from entering and with which activity due to atomization increases to the limit, for example, 0.5 to 1000 µm, preferably 1 to 500 µm, further preferably 1 to 250 µm, and most preferably 1 to 150 µm.

Reversely, by making the microparticles of metal or metal hydride and precious metal microparticles into colloids, particle diameter may be reduced to a size that allows permeation through the skin, resulting in reduction of the object of antioxidation in a region deeper than the skin. The description of the aforementioned preferable particle diameter of precious metal microparticles may be referred to in order to obtain such particle diameter. In any case, the present invention may include various particle diameters of the impalpable powder of metal or metal hydride or precious metal microparticles.

According to another embodiment, an application-type external medicine for treatment or prevention may be considered.

As a concrete example thereof, a layer containing zinc powder is made to be a first adhesive layer, which is to make contact with skin, and a second adhesive layer containing a citric acid solution or citric acid solution containing platinum microparticles is placed on that first layer via a plastic plate. Once the application-type external medicine for treatment or prevention made up of this structure is applied on the object of antioxidation or target region of skin or a mucous membrane and the plastic plate is pulled out, the first layer and the second layer make contact, generating active hydrogen or activating hydrogen molecules, as well as supplying electrons to the object of antioxidation at the same time.

Note that at this time, in order to prevent dispersion and loss of the generated active hydrogen or hydrogen molecules into the air, it is desirable that the second adhesive layer is covered with a cover made of a material such as aluminum that will keep in the hydrogen molecules. Needless to say, a structure in which the first adhesive layer may be the top layer and the second adhesive layer the lower layer may be used. Moreover, platinum microparticles may be included in the first adhesive layer.

Zinc has been exemplified here because, as described later, generation of active hydrogen or activation of hydrogen molecules due to zinc is more gradual than with a metal such as magnesium on one hand; however, such gradualness is assumed to be preferable for such a form as an application agent that preferably releases an active ingredient over time. However, this exemplification does not limit available active hydrogen generating agent or metal used in the application agent according to the present invention.

According to yet another embodiment, a moxa cautery-type external medicine for treatment or prevention may be considered.

As a concrete example, a citric acid solution or citric acid solution containing platinum microparticles is applied on the object of antioxidation or target region of skin or a mucous membrane, and powder containing metal magnesium impalpable powder is heaped on top to build a mound of the equivalent size of a fingertip. Thereby, the citric acid solution reacts with the metal magnesium impalpable powder, generating active hydrogen or activating hydrogen molecules, as well as supplying electrons to the object of antioxidation at the same time. Alternatively, it may have a structure where a citric acid solution or citric acid solution is applied on the object of antioxidation or target region of skin or a mucous membrane, and a powder containing metal magnesium impalpable powder and a powder further containing platinum microparticles as needed is heaped on top to build a mound of the equivalent size of a fingertip. Further alternatively, it may have a structure where a metal magnesium impalpable powder and a powder containing citric acid and powder platinum microparticles as needed are heaped on the object of antioxidation or target region of skin or a mucous membrane to build a mound of the equivalent size of a fingertip, and then a moderate quantity of a solvent is dripped thereupon.

According to yet another embodiment, use of an oral-type external medicine for treatment or prevention may be considered. As a concrete example, an oral drug containing metal magnesium impalpable powder and precious metal microparticles such as platinum microparticles as needed is administered to the object of antioxidation or target region of skin or a mucous membrane. Thereby, gastric juice or acid solution reacts with the metal magnesium impalpable powder, generating active hydrogen or activating hydrogen molecules, as well as supplying electrons to the object of antioxidation at the same time. It is desirable that such oral drug is properly coated with a gastrosoluble capsule or the like.

### [Working Examples]

Working examples of the present invention are described next.

[Keeping quality evaluation test through coloration change of methylene blue]

### [Working Example 1]

First agent: Metal magnesium powder (particle diameter: 212 to 600 µm, 99.9%) (reagent manufactured by Wako Pure Chemical Industries, Ltd.) is used as a first agent.

Second agent: A solution resulting from 3 g of citric acid (C₆H₈O₇ manufactured by Wako Pure Chemical Industries, Ltd.) added to a liquid, which is made of 0.05 g of a 4 wt% platinum colloid solution manufactured by Tanaka Kikinzoku dissolved in 100 mL distilled water manufactured by Wako Pure Chemical Industries, Ltd., is used as a second agent.

The first agent and the second agent are stored in separate plastic containers

72 hours later, 0.5 mL of a 1g/L concentration methylene blue (tetramethylthionine chloride; C₁₆H₁₈ClN₃S•3(H₂O), manufactured by Wako Pure Chemical Industries, Ltd.) solution (hereafter referred to as MB solution) is dripped on a plate, and 0.01 g of the first agent and 0.5 mL of the second agent are then dripped thereupon in this order.

After the second agent is dripped, duration until the blue color of the MB solution on the plate disappears is measured.

### [Comparative Example 1]

Hydrogen gas is bubbled into activated carbon treated water, and a solution, which results from adding 0.05 g of the 4 wt% platinum colloid solution manufactured by Tanaka Kikinzoku to 100 mL hydrogen-dissolved water in which hydrogen molecules are dissolved to a saturated concentration (hereafter referred to as antioxidant water), is created and stored in a plastic container.

72 hours later, 0.5 mL of a 1 g/L concentration methylene blue (tetramethylthionine chloride; C₁₆H₁₈CIN₃S•3(H₂O) solution (hereafter referred to as MB solution) is dripped on a plate, and 0.5 mL of the antioxidant water is then dripped thereupon.

After the antioxidant water is dripped, duration until the blue color of the MB solution on the plate disappears is measured.

Results thereof are given in Table 1 below.

**[Table 1]**

| | |
|---|---|
| Working Example 1 | 5 seconds |
| Comparative Example 1 | does not disappear |
| [Keeping quality evaluation test through coloration change of DPPH radicals] | |
| [Working Example 2] | |

First agent: Metal magnesium powder (particle diameter: 212 to 600 µm, 99.9%) (reagent manufactured by Wako Pure Chemical Industries, Ltd.) is used as a first agent.

Second agent: A solution resulting from 3 g of citric acid (C₆H₈O₇ manufactured by Wako Pure Chemical Industries, Ltd.) added to a liquid, which is made of 0.05 g of a 4 wt% platinum colloid solution manufactured by Tanaka Kikinzoku dissolved in 100 mL distilled water manufactured by Wako Pure Chemical Industries, Ltd., is used as a second agent.

The first agent and the second agent are stored in separate plastic containers.

72 hours later, 1 mL of a 0.16 g/L concentration DPPH manufactured by Calbioche (1, 1-diphenyl-2-piccrylhydrazyl) solution (hereafter referred to as DPPH solution) is put into a measuring cylinder, and 0.01 g of the first agent and 0.5 mL of the second agent are then dripped thereupon in this order.

After the second agent is dripped, duration until the dark red color of the DPPH solution on the plate fades is measured.

### [Comparative Example 2]

Antioxidant water is made and stored in a plastic container.

72 hours later, 1 mL of the DPPH solution is put into a measuring cylinder, and 0.5 mL of the antioxidant water is then dripped thereupon.

After the antioxidant water is dripped, duration until the dark red color of the DPPH solution on the plate fades is measured.

Results thereof are given in Table 2 below.

**[Table 2]**

| | |
|---|---|
| Working Example 2 | 60 seconds |
| Comparative Example 2 | does not fade |

### [Observation of Working Examples]

The methylene blue takes on a blue color when in an oxidizing form, while when reduced, it forms reduced methylene blue (leucomethylene blue) and the blue color disappears. Therefore, if the methylene blue in an oxidizing environment is exposed to an arbitrary reagent and the blue color disappears thereafter, that reagent may be taken to have reducing power.

Similarly, the deep red color of the DPPH radical fades when reduced, and thus if it is exposed to an arbitrary reagent and the deep red color fades thereafter, that reagent may be taken to have radical eliminating activity or antioxidation power.

In Working Examples 1 and 2 given above, the external medicine for treatment or prevention according to the present invention made the blue color of the methylene blue disappear and the deep red color of the DPPH radical fade even after being stored for 72 hours in a plastic container; however, on the other hand, the antioxidant water of Comparative Examples 1 and 2 did not make the blue color of the methylene disappear nor the deep red color of the DPPH radical fade.

The antioxidant water containing hydrogen molecules and platinum microparticles is capable of reducing methylene blue or DPPH radical, which are subjects of antioxidation, right after it is made. However, under the condition of being stored for a long period in a plastic container, since the hydrogen molecules in the container are gradually dispersed and lost into the air, and the hydrogen molecules react with an oxide such as oxygen that penetrates in from the air via the platinum microparticles or catalyst, by the time it meets the object of antioxidation or target (methylene blue or DPPH radical) after 72 hours, there is hardly any of the hydrogen molecules left. On the other hand, with the external medicine for treatment or prevention according to the present invention, keeping quality of the hydrogen molecules, which are substances that are very difficult to store essentially, is irrelevant.

Moreover, with the antioxidant water, since there is such a constraint that hydrogen molecules must be included beforehand in the water or solvent, a greater quantity of hydrogen molecules than the saturation solubility in the solvent cannot be utilized. The solubility of hydrogen molecules into water is only 1.6 mg/L at 20 degrees Celsius at 1 atm, which is too low to have a significant influence on an organism.

Meanwhile, with the external medicine for treatment or prevention of this specification, since hydrogen molecules are not included beforehand in the solvent, there is no temporal dispersion and loss of the hydrogen molecules into the air, nor wasteful consumption of hydrogen molecules due to an oxidant other than the target, and a greater quantity of hydrogen molecules than the saturation solubility in the solvent may be utilized. Since generation of active hydrogen or activation of hydrogen molecules and consumption thereof in the object of antioxidation occur at the same time, utilization of the antioxidation power of the active hydrogen is possible immediately at any time.

As such, the external medicine for treatment or prevention according to the present invention has many merits that cannot be attained only by simply using the antioxidant water as the external medicine for treatment or prevention.

[Reducing power evaluation test through coloration change of methylene blue]

### 1. Test procedure

As a basic test procedure, first prepare 1 g of each test article, perform operation for generating hydrogen for those that require it, and drip thereupon a methylene blue aqueous solution adjusted to a constant concentration. If these test articles have reducing power, the dripped blue methylene blue will be reduced and turn colorless; however, if quantity of the methylene blue aqueous solution poured in is gradually increased, and the reducing agent within the test articles is completely consumed, coloration change of the methylene blue from blue to colorless cannot be observed. Reducing power of each of the test articles is evaluated from the total dripped quantity of methylene blue aqueous solution until this time.

A specific testing method is described next.

As methylene blue aqueous solutions to be dripped, prepare a 50 mg/L (volume molarity: 156.3 µM) one, a 1 g/L (volume molarity: 3126.5 µM), and a 2.5 g/L (volume molarity: 7816.3 µM) one. Here, three types of methylene blue aqueous solutions differing in concentration are prepared because since there is difference in reducing power among the test articles, higher test accuracy may be expected if three types of concentration of methylene blue are properly used according to reducing power given to the respective test articles.

Next, in the environment when methylene blue is actually dripped, since hydrogen activated on the surface of precious metal microparticles or catalyst reduces the methylene blue, and at the same time, and reacts with the oxygen in the solution or the air and is consumed, testing should be conducted essentially in an enclosed environment using test articles expelled of any existing air.

However, this test is in a different situation. Supposing that an enclosed environment is created by plugging test tubes with rubber plugs, and generation and reaction of hydrogen and dripping of the methylene blue aqueous solutions is performed in that environment, in the case of making, for example, calcium and water react hydrogen is generated vigorously, the rubber plugs will be blown off, and the enclosed environment cannot be maintained. This is something not limited to calcium, and can be said about other several test articles to which this test is conducted. Moreover, empirically, as a result of considering that impact (approximately several ppm) of oxygen in the reagent and impact due to reaction with the oxygen in the air upon comparison among the test articles is not a big problem, it has been decided to conduct this test in air instead of doing the same in an oxygen-free environment.

20 mL test tubes are used as the containers. Place 1 g of the test article in each of the test tubes, and drip 1 mL of the respective methylene blue aqueous solutions therein using a pipet. Shake and stir the test tubes each time 1 mL is dripped in while visually observing for any color reaction. Note that the color reaction of methylene blue is reversible and dripping of methylene blue must be performed quickly since even methylene blue that has been reduced once to be colorless returns to a blue color if oxidized in air.

### 2. Presentation of working examples, comparative examples, and reference examples

### [Working Example 3]

50 mg of magnesium powder (-100 + 200 mesh, 99.6%) manufactured by Alfa Aesar (ALF) is used as a first agent, and 950 mg of a solution resulting from diluting two-hundredfold purified water, which is Fujisawa City tap water processed through an ion-exchange column manufactured by Organo Corporation, with a 4 wt% platinum colloid solution (200 mg/L platinum colloid concentration, the same is used hereafter) manufactured by Tanaka Kikinzoku Kogyo K.K., and adding citric acid to 20% concentration (200 g/L, the same is used hereafter) is used as a second agent.

A mixture of the first agent and the second agent is used as a test article. After adjustment of the test article, 1 mL of each of the methylene blue aqueous solutions is dripped thereupon while visually observing color change of the test article. Quantity of the methylene blue aqueous solutions dripped until color change is no longer confirmed is 6 mL for a solution of 2.5 g/L concentration, and 3 mL for that of 1 g/L concentration, and total reduced methylene blue is 56.3 µmol.

### [Working Example 4]

50 mg of zinc powder (particle diameter: approximately 6 µm) manufactured by Wako Pure Chemical Industries, Ltd. is used as a first agent, and 950 mg of a solution resulting from diluting two-hundredfold purified water, which is Fujisawa City tap water processed through an ion-exchange column manufactured by Organo Corporation, with a 4 wt% platinum colloid solution manufactured by Tanaka Kikinzoku Kogyo K.K., and adding citric acid to 20% concentration is used as a second agent.

A mixture of the first agent and the second agent is used as a test article. After adjustment of the test article, 1 mL of each of the methylene blue aqueous solutions is dripped thereupon while visually observing color change of the test article. Quantity of the methylene blue aqueous solutions dripped until color change is no longer confirmed is 12 mL for a solution of 2.5 g/L concentration, and 2 mL for that of 1 g/L concentration, and total reduced methylene blue is 100.0 µmol.

### [Working Example 5]

50 mg of calcium granules manufactured by Wako Pure Chemical Industries, Ltd. is used as a first agent, and 950 mg of a solution resulting from diluting two-hundredfold purified water, which is Fujisawa City tap water processed through an ion-exchange column manufactured by Organo Corporation, with a 4 wt% platinum colloid solution manufactured by Tanaka Kikinzoku Kogyo K.K. is used as a second agent.

A mixture of the first agent and the second agent is used as a test article. After adjustment of the test article, 1 mL of each of the methylene blue aqueous solutions is dripped thereupon while visually observing color change of the test article. Quantity of the methylene blue aqueous solutions dripped until color change is no longer confirmed is 6 mL for a solution of 2.5 g/L concentration, and total reduced methylene blue is 46.9 µmol.

### [Working Example 6]

50 mg of sodium borohydride manufactured by Merck & Co., Inc. is used as a first agent, and 950 mg of a solution resulting from diluting two-hundredfold purified water, which is Fujisawa City tap water processed through an ion-exchange column manufactured by Organo Corporation, with a 4 wt% platinum colloid solution manufactured by Tanaka Kikinzoku Kogyo K.K. is used as a second agent.

A mixture of the first agent and the second agent is used as a test article. After adjustment of the test article, 1 mL of each of the methylene blue aqueous solutions is dripped thereupon while visually observing color change of the test article. Quantity of the methylene blue aqueous solutions dripped until color change is no longer confirmed is 6 mL for a solution of 2.5 g/L concentration, and total reduced methylene blue is 46.9 µmol.

### [Working Example 7]

50 mg of calcium hydride manufactured by Wako Pure Chemical Industries, Ltd. is used as a first agent, and 950 mg of a solution resulting from diluting two-hundredfold purified water, which is Fujisawa City tap water processed through an ion-exchange column manufactured by Organo Corporation, with a 4 wt% platinum colloid solution manufactured by Tanaka Kikinzoku Kogyo K.K. is used as a second agent. As in the other examples, mixing the first agent and the second agent and dripping methylene blue was attempted; however, since reaction between the first agent and the second agent was extremely severe, and while the first agent and the second agent generate hydrogen at the instant they make contact, water spatters and the reaction instantaneously ends, measurements could not be taken.

### [Working Example 8]

50 mg of magnesium powder ((-100 + 200 mesh, 99.6%) manufactured by Alfa Aesar (ALF) is used as a first agent, and 950 mg of a solution resulting from diluting two-hundredfold purified water, which is Fujisawa City tap water processed through an ion-exchange column manufactured by Organo Corporation, with a 4 wt% palladium colloid solution manufactured by Tanaka Kikinzoku Kogyo K.K., and adding citric acid to 20% concentration is used as a second agent.

A mixture of the first agent and the second agent is used as a test article. After adjustment of the test article, 1 mL of each of the methylene blue aqueous solutions is dripped thereupon while visually observing color change of the test article. Quantity of the methylene blue aqueous solutions dripped until color change is no longer confirmed is 4 mL for a solution of 2.5 g/L concentration, and 3 mL for that of 1 g/L concentration, and total reduced methylene blue is 40.3 µmol.

### [Working Example 9]

50 mg of magnesium powder ((-100 + 200 mesh, 99.6%) manufactured by Alfa Aesar (ALF) is used as a first agent, and 950 mg of a solution resulting from adding citric acid to purified water, which is Fujisawa City tap water processed through an ion-exchange column manufactured by Organo Corporation, to 20% concentration is used as a second agent.

A mixture of the first agent and the second agent is used as a test article. After adjustment of the test article, 1 mL of each of the methylene blue aqueous solutions is dripped thereupon while visually observing color change of the test article. Quantity of the methylene blue aqueous solutions dripped until color change is no longer confirmed is 1 mL for a solution of 50 g/L concentration, and total reduced methylene blue is 0.2 µmol.

### [Comparative Example 3]

1 mL of a solution resulting from adding 4 wt% platinum colloid solution manufactured by Tanaka Kikinzoku Kogyo K.K. to saturated hydrogen water, which results from bubbling hydrogen gas for 90 minutes into purified water or Fujisawa City tap water processed through an ion-exchange column manufactured by Organo Corporation to a 200 mg/L platinum colloid concentration, is used as a test article. After adjustment of the test article, 1 mL of each of the methylene blue aqueous solutions is dripped thereupon while visually observing color change of the test article. Quantity of the methylene blue aqueous solutions dripped until color change is no longer confirmed is 2 mL for a solution of 50 g/L concentration, and total reduced methylene blue is 0.3 µmol.

### [Reference Example 1]

1 mL of saturated hydrogen water, which results from bubbling hydrogen gas for 90 minutes into purified water or Fujisawa City tap water processed through an ion-exchange column manufactured by Organo Corporation, is used as a test article. After adjustment of the test article, 1 mL of each of the methylene blue aqueous solutions is dripped thereupon while visually observing color change of the test article. 1 mL of the methylene blue aqueous solution with the weakest concentration of 50 mg/L could not be turned transparent.

### [Reference Example 2]

1 mL of a solution resulting from diluting two-hundredfold purified water, which is Fujisawa City tap water processed through an ion-exchange column manufactured by Organo Corporation, with a 4 wt% platinum colloid solution manufactured by Tanaka Kikinzoku Kogyo K.K., and adding citric acid to 20% concentration is used as a test article. After adjustment of the test article, 1 mL of each of the methylene blue aqueous solutions is dripped thereupon while visually observing color change of the test article. 1 mL of the methylene blue aqueous solution with the weakest concentration of 50 mg/L could not be turned transparent.

### [Reference Example 3]

1 mL of a solution resulting from diluting two-hundredfold purified water, which is Fujisawa City tap water processed through an ion-exchange column manufactured by Organo Corporation, with a 4 wt% platinum colloid solution manufactured by Tanaka Kikinzoku Kogyo K.K., is used as a test article. After adjustment of the test article, 1 mL of each of the methylene blue aqueous solutions is dripped thereupon while visually observing color change of the test article. 1 mL of the methylene blue aqueous solution with the weakest concentration of 50 mg/L could not be turned transparent. 3. Test results

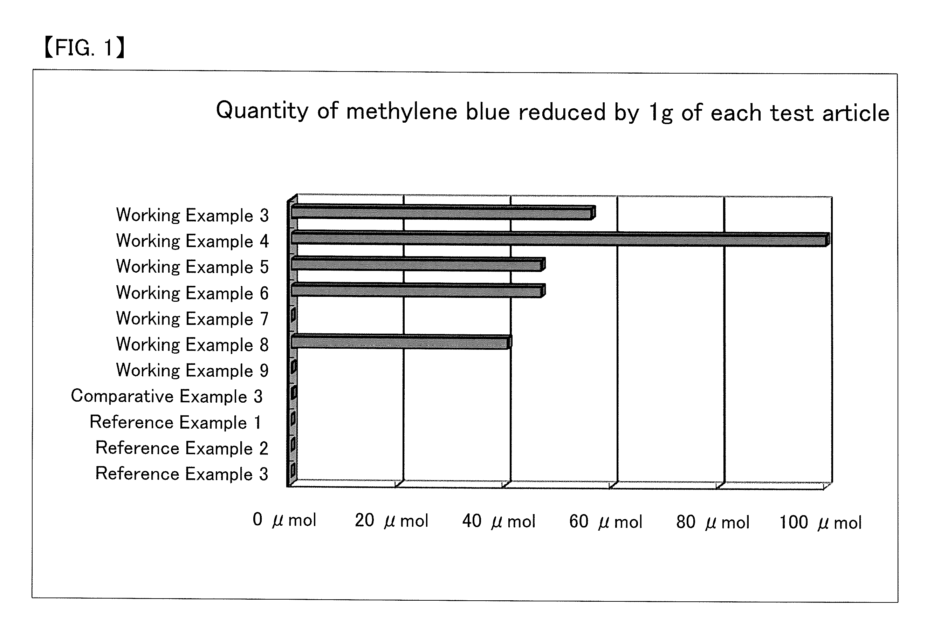

Test results are given in a graph of FIG. 1 and Table 3 below.

**[Table 3]**

| Test Article Name | Content | Quantity of methylene blue reduced by 1g of each test article |
|---|---|---|
| Working Example 3 | Magnesium + platinum colloid + citric acid | 56.3 µmol |
| Working Example 4 | Zinc + platinum colloid + citric acid | 100.0 µmol |
| Working Example 5 | Calcium + platinum colloid | 46.9 µmol |
| Working Example 6 | Sodium borohydride + platinum colloid | 46.9 µmol |
| Working Example 7 | Calcium hydride + platinum colloid | Incapable measurement |
| Working Example 8 | Magnesium + palladium colloid + citric acid | 40.6 µmol |
| Working Example 9 | Magnesium + citric acid | 0.2 µmol |
| Comparative Example 3 | Saturated hydrogen water + platinum colloid | 0.3 µmol |
| Reference Example 1 | Saturated hydrogen water | 0.0 µmol |
| Reference Example 2 | Platinum colloid + citric acid | 0.0 µmol |
| Reference Example 3 | Platinum colloid | 0.0 µmol |

### 4. Observation of test results

While active hydrogen may be considered to be fundamentally very unstable, since the external medicine for treatment or prevention generates active hydrogen or activates hydrogen molecules in a state of being in contact with the object of antioxidation, which is an affected part, the reducing power of the active hydrogen may effectively act on the affected part while confining the active hydrogen in the affected part (providing viscosity to the agent by a dispersant is preferred.)

In this test, the external medicine using zinc as the first agent in Working Example 4 has reduced the largest quantity of methylene blue. Upon visual confirmation during the test, generation rate of hydrogen gas bubbles is very gradual in Working Example 4. As a result, in Working Example 4, it is believed that the generated hydrogen molecules have been effectively used for reduction of the methylene blue without any of the generated hydrogen molecules escaping into the air.

On the contrary, in Working Example 5, which has used calcium for the first agent, Working Example 6, which has used sodium borohydride for the first agent, and Working Example 7, which has used calcium hydride for the first agent, very high reactivity has been exhibited, and reaction with the neutral water has been vigorous even without adding citric acid. Among them, since the calcium hydride of Working Example 7 has reacted strongly just by touching the water surface, data could not be acquired through this testing method.

### [Antioxidative activity evaluation test through coloration change of DPPH radicals]

### 1. Test procedure

This test is carried out according to the test procedure of the reducing power evaluation test through coloration change of methylene blue described above. Namely, 1 cc of the test article and 1 cc of the DPPH solution are placed in a cell, and the DPPH solution in the cell is visually checked whether or not it turns to amber. If the DPPH in the cell changes from purple to amber, this means that the test article would have reduced the DPPH radicals in the solution.

In the case where the test article has reduced the DPPH radicals, concentration of the DPPH solution is gradually increased, and antioxidation power among the test articles is compared from the DPPH concentration until the point where change to amber color can no longer be confirmed. Since the hydrogen reacts with the oxygen in the solution and in the air when there is a catalyst, under ordinary circumstances, testing should be conducted after making the cell interior or reagent oxygen-free through replacement by nitrogen gas; however, since a large quantity of hydrogen generates in the cell due to the property of the hydrogen generating agent, a lid cannot be attached, and thereby testing is conducted in a state where oxygen is not removed, and there is no lid on the cell. The same conditions are used for the comparative examples in order to unify the conditions.

Note that DPPH manufactured by Calbioche is used, and ethanol manufactured by Wako Pure Chemical Industries, Ltd. is used as the solvent. Concentrations are increased from 25 mg/L to 50 mg/L to 100 mg/L to 200 mg/L to 400 mg/L, and to 500 mg/L, and then continuing to further increase in units of 100 mg/L until 5000 mg/L, which is the maximum concentration of DPPH radicals that may be dissolved in ethanol.

### 2. Presentation of working examples, comparative examples, and reference examples

### [Working Example 10]

50 mg of magnesium powder ((-100 + 200 mesh, 99.6%) manufactured by Alfa Aesar (ALF) is used as a first agent, and 950 mg of a solution resulting from diluting two-hundredfold purified water, which is Fujisawa City tap water processed through an ion-exchange column manufactured by Organo Corporation, with a 4 wt% platinum colloid solution manufactured by Tanaka Kikinzoku Kogyo K.K., and adding citric acid to 20% concentration is used as a second agent.

A mixture of the first agent and the second agent is used as a test article. After adjustment of the test article, the DPPH solutions are dripped in order from lowest concentration until color change can no longer be confirmed. Color changed could be confirmed up to 5000 mg/L, which is the maximum concentration.

### [Comparative Example 4]

1 mL of a solution resulting from adding a 4 wt% platinum colloid solution manufactured by Tanaka Kikinzoku Kogyo K.K. to saturated hydrogen water, which results from bubbling hydrogen gas for 90 minutes into purified water or Fujisawa City tap water processed through an ion-exchange column manufactured by Organo Corporation to a 200 mg/L platinum colloid concentration, is used as a test article. After adjustment of the test article, the DPPH solutions are dripped in order from the lowest concentration until color change can no longer be confirmed. Ultimately, the concentration of the DDPH solution with which a changed color is confirmed is 200 mg/L.

### 3. Test results

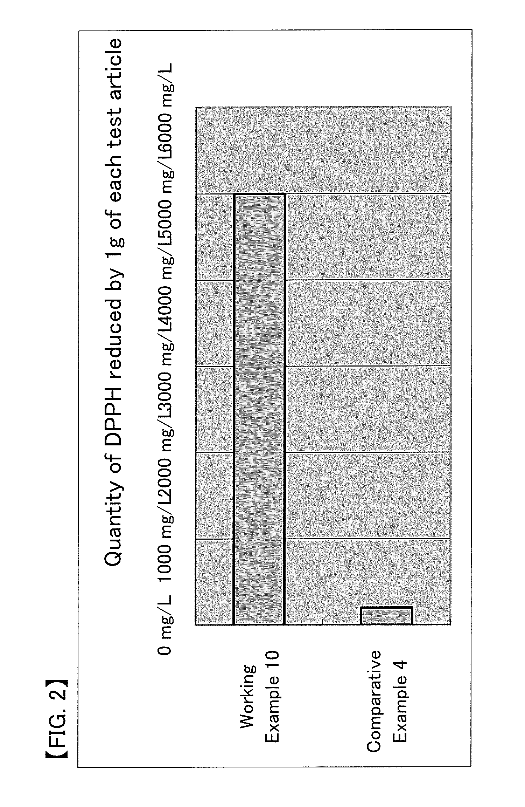

Test results of the above-given tests are given in a graph of FIG. 2 and Table 4.

**[Table 4]**

| | Quantity of DPPH reduced by 1g of each test article |
|---|---|
| Working Example 10 | 5000 mg/L |
| Comparative Example 4 | 200 mg/L |

### 4. Observation of test results

With this test method, while the concentration of DDPH that has been reduced in Working Example 10 is 5000 mg/L or maximum solubility, reduction of DPPH with an extremely high concentration should be potentially possible. Theoretically, while 50 mg of magnesium generates 4.17 mg of hydrogen, this is around 2600 times the hydrogen quantity of approximately 0.0016 mg contained in 1 g of saturated hydrogen water at 20 degrees Celsius at 1 atm.

Note that quantity of hydrogen generated from the hydrogen generating agent used for the external medicine for treatment or prevention in this specification may be calculated in the following manner. For example, in the case of using reaction between metal magnesium and water as the hydrogen generating agent, it can be understood from the reaction formula Mg + 2H2O → Mg(OH)2 + H2 that 1 mol (2 g) of hydrogen generates from 1 mol (24 g) of magnesium. Here, assuming preparation of 1 L of the external medicine for treatment or prevention, it may be constituted by 200 g of the first agent and 800 g of the second agent in a structural ratio of 1:4, for example, but the present invention is not limited thereto.

If concentration of the metal magnesium included in the first agent is 5 wt%, for example, the quantity consumed of the metal magnesium is 10 g. As described above, in this working example, since it can be seen that 1 mol (2 g) of hydrogen generates from 1 mol (24 g) of magnesium, quantity of hydrogen to be generated from 10 g of metal magnesium may be calculated to be around 830 mg. While this number is merely a calculated value, taking into consideration the fact that only 1.6 mg/L can be dissolved in the case where hydrogen molecules are to be dissolved in water at 20 degrees Celsius at 1 atm, as described above, for example, advantage thereof is apparent. Moreover, in the case of making a catalyst coexist with such hydrogen-dissolved water, since dissolved oxide such as oxygen reacts with the hydrogen once the catalyst is made to coexist therein, needless to say, quantity of actually available hydrogen at the time of use is further decreased.

Note that while the aforementioned calculated value assumes that all of the metal magnesium reacts with a protic solvent such as water, acid solution, or the like, actual measurement of generated hydrogen quantity is assumed to shift from the calculated value due to particle diameter of the metal magnesium and thickness of the passive film covering the surface thereof. Therefore, actual measurement of generated hydrogen quantity is measured by the conventional water replacement method.

As a result, 5 mL of hydrogen is generated in the case where 30 g of purified water is made to react with 0.5 g of metal magnesium (particle diameter of 150 µm or less), and 280 to 485 mL of hydrogen in the case where 30 g of a 5 to 10 wt% citric acid solution is made to react with. If these values are converted, generated hydrogen quantity for 10 g of metal magnesium is 17.9 mg from the reaction with purified water, and 500 to 866 mg from the reaction with the citric acid solution.

### [Preparation method of external medicine for treatment or prevention]

When actually trying to prepare the external medicine for treatment or prevention of the present invention, there may be several forms thereof such as pill, patch, and the like; however, a composition example of when preparing a gel external medicine is given here. Note that this composition example is not intended to limit the present invention.

With the premise that one of a two-agent composition is a first agent, and the other is a second agent, and 2 parts of the second agent is mixed to 1 part of the first agent, the respective compositions are as follows, for example.

### First agent:

5.0 wt% magnesium powder MG 100 (50 µm or less) (Kanto Metal Corporation)
2.0 wt% Metolose (methylcellulose) SNB-30T (Shin-Etsu Chemical Co., Ltd.)
10.0 wt% glycerin (TOHO Medicine Manufacturing Co. Ltd.)
0.05 wt% Calfa (grapefruit seed extract, preservative) (Calfa Chemical Co., Ltd.)
82.95 wt% purified water

### Second agent:

0.32 wt% (concentration of platinum colloid is 128 mg/L) of 4 wt% platinum colloid solution (Tanaka Kikinzoku Kogyo K.K.)
10.0 wt% citric acid (Wako Pure Chemical Industries, Ltd.)
0.5 wt% hydroxypropylcellulose (Nippon Soda Co., Ltd.)
5 wt% glycerin (TOHO Medicine Manufacturing Co. Ltd.)
0.05 wt% Calfa (grapefruit seed extract, preservative) (Calfa Chemical Co., Ltd.)
84.13 wt% purified water
While the magnesium powder of the first agent is dispersed uniformly in the gel, this dispersion is only physically maintained by the viscosity of the water. Since the specific gravity of magnesium is greater, the magnesium will gradually sink if the viscosity is weak, and therefore a stable product cannot be provided. To this effect, smaller particle diameter of the magnesium powder is better. If possible, a particle diameter in the vicinity of 150 µm used in this example or smaller is preferred. Moreover, the viscosity dispersants of any types and concentrations used here have to be capable of stably maintaining the magnesium in the gel. Accordingly, further addition of an antisettling agent such as polyethylene oxide, amide wax, or dry silica as needed is preferred.

Furthermore, platinum colloid is employed as a precious metal microparticle catalyst in the aforementioned composition of the second agent; however, the present invention is naturally not limited to platinum colloid, and as given in a part of the working examples, precious metal microparticles such as palladium colloid or gold colloid, alloy compound or metal complex thereof, and hydrogen oxidation-reduction enzyme are also candidates.

Note that it is preferred that the citric acid in the composition of the second agent is included in a certain concentration capable of dissolving all of the magnesium powder within the first agent when mixed with the first agent. In the composition example given above, the first agent is alkaline in the vicinity of pH 11, and the second agent is strong acidity of slightly beyond pH 2. When mixing these agents at a ratio of 1:2, the pH is approximately 3, which is acidic. When the first agent and the second agent are mixed together, the passive film formed on the magnesium surface in the first agent is broken due to the citric acid of the second agent, which begins the reaction of:

### <Formula 8>

Mg + 2H₂O → Mg(OH)₂ + H₂

The pH that was approximately 3 right after mixing is pulled toward the alkali direction due to this reaction (action of hydroxide ions). At this time, if the acid in the second agent is insufficient, before all of the magnesium in the first agent is consumed, it turns into an environment in a range from neutral to alkali, and the magnesium that has not been consumed up to that point forms another passive film and stops reacting. As a result, magnesium powder is left, which is undesirable as far as convenience is concerned. Note that in this example, the citric acid concentration is sufficient, and pH after all of the magnesium has reacted is around 4, which is acidic.

Generally speaking, when using a substance that generates a hydroxyl group from reaction with a supporting agent such as metal magnesium as a first agent, the first agent has a pH of 7 to 14, more specifically 9 to 12, even further specifically in the vicinity of 11, which indicates alkalinity, and the second agent, which aims to neutralize the alkalinity of the first agent, neutralize the alkalinity that results from the reaction between the first agent and the second agent, and supply a proton to the system, has a pH of 1 to 7, more specifically 1 to 4, even further specifically 1 to 3, and most specifically in the vicinity of 2, which indicates mild acidity. Even if it is after the first agent and the second agent are mixed and reaction is completed, for example, once 72 hours have elapsed after mixing, it is preferable that liquidity of the compound liquid maintains mild to normal acidity due to the acid of the second agent.

### [Dermatitis inhibition test for atopic dermatitis model mouse]

### 1. Test procedure

### 1.1 Manufacturing of atopic dermatitis model (initial outbreak)

Once hair on the back and auricles of a NC/Nga mouse is shaved using a hair clipper or electric shaver, an appropriate amount of a depilatory agent (Product name: Epilat, manufactured by Kanebo Cosmetics Inc.) is applied thereto.

Once the depilatory agent is wiped off, 100 mg of Biostir AD (manufactured by Biostir Inc.), which is an ointment for dermatophagoides farinae-induced atopic dermatitis, is applied uniformly onto the back and auricles using the back of a micropipette tip.

### 1.2 Preparation of atopic dermatitis model (second and subsequent outbreaks)

After hair removal using a shaver as needed, 150 µl of 4% sodium dodecylsulfate aqueous solution is applied uniformly onto the back and auricles using the back of a micropipette tip while dripping it thereupon.

Afterward, once it is dried to a certain degree using a hair dryer (cold air) and dry naturally for approximately 2 to 3 hours, 100 mg of Biostir AD is then applied onto the back and auricles using the back of a micropipette tip.

* All processing is carried out twice a week. Atopic dermatitis mice are prepared through processing for 3 weeks, a total of 6 times.

### 1.3 Administration to test articles

The mice are divided into groups of six so that dermatitis severity is uniform, and 100 mg of the solution is applied uniformly on the skin of auricles and back once a day.

### 1.4 Check dermatitis severity

Scores are given visually based on the dermatitis severity score table of Section 1.5. Scores are checked on 0, 3, 6, 9, 12, 15 ... days after having started administration of the test articles.

### 1.5 Dermatitis severity score table

### (a) Reddening and bleeding

(Reddening and bleeding symptoms on the back are observed)
0: No symptoms; no reddening or bleeding symptoms on the back
1: Light; reddening locally on the back, but no bleeding associated with continuous abrasions
2: Moderate; reddening sporadically on the back, and no bleeding associated with continuous abrasions
3: Serious; reddening across the entire back, or bleeding associated with continuous abrasions

### (b) Incrustation and dryness

(Incrustation and dryness symptoms on the back are observed)
0: No symptoms; no incrustation or dryness symptoms on the back
1: Light; Slight whitening of the skin, and slight peeling of keratin on the back locally
2: Moderate; Apparent peeling of keratin on the back locally
3: Serious; crusta across the entire back, and apparent peeling of keratin

### (c) Edema

(Edema of auricle is observed qualitatively)
0: No symptoms; no thickness in both auricles
1: Light; slight thickness in one of the auricles
2: Moderate; Apparent thickness and tension in either auricle
3: Serious; apparent thickness, tension, and curvature in each auricle, and hardness is felt when touched with a finger

### (d) Abrasion and tissue deficit

(Abrasion and tissue deficit symptoms of the auricles are observed)
0: No symptoms; no abrasions or tissue deficit symptoms of the auricles
1: Light; non-continuous abrasions in the auricles but no tissue deficit
2: Moderate; small continuous abrasions in the auricles but no tissue deficit
3: Serious; continuous abrasions and tissue deficit in the auricles
2. Presentation of working examples, comparative examples, and reference examples

### [Working Example 11]

First agent: A composite constituted by 5.0 wt% of magnesium powder MG 100 (150 µm or less) manufactured by Kanto Metal Corporation, 2.0 wt% of Metolose (methylcellulose) SNB-30T manufactured by Shin-Etsu Chemical Co., Ltd., 10.0 wt% of Glycerin manufactured by TOHO Medicine Manufacturing Co. Ltd., 0.05 wt% of Calfa manufactured by Calfa Chemical Co., Ltd., and 82.95 wt% of purified water, which is Fujisawa City tap water processed through an ion-exchange column manufactured by Organo Corporation.

Second agent: A composite constituted by 0.32 wt% of 4 wt% platinum colloid solution manufactured by Tanaka Kikinzoku Kogyo K.K., 10.0 wt% of citric acid manufactured by Wako Pure Chemical Industries, Ltd., 0.5 wt% of hydroxypropylcellulose manufactured by Nippon Soda Co., Ltd., 5.0 wt% of Glycerin manufactured by TOHO Medicine Manufacturing Co. Ltd., 0.05 wt% of Calfa manufactured by Calfa Chemical Co., Ltd., and 84.13 wt% of purified water, which is Fujisawa City tap water processed through an ion-exchange column manufactured by Organo Corporation.

When applying to a model animal, mix the first agent and the second agent in a weight ratio of 1:2, and immediately apply 100 mg of the foaming creamy mixture.

Notation 'Test article group HM031' is given in the tables and graphs.

### [Comparative Example 5]

By mixing the first agent and the second agent of Working Example 11 and leaving it for at least 72 hours, the reaction product from which hydrogen has been removed is presumed as the base in Working Example 11 and 100 mg thereof is applied.

### Notation 'Medium subject group control' is given in the tables and graphs.

### 3. Test results

### 3.1 Check dermatitis severity

Changes in dermatitis scores in the dermal administration test of Working Example 11 are shown categorized into whole area (back + auricles), back, and auricles.

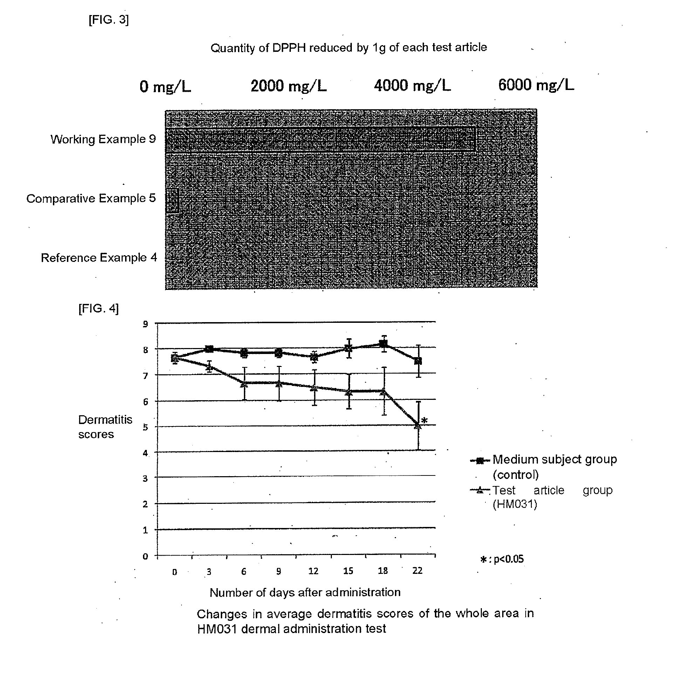

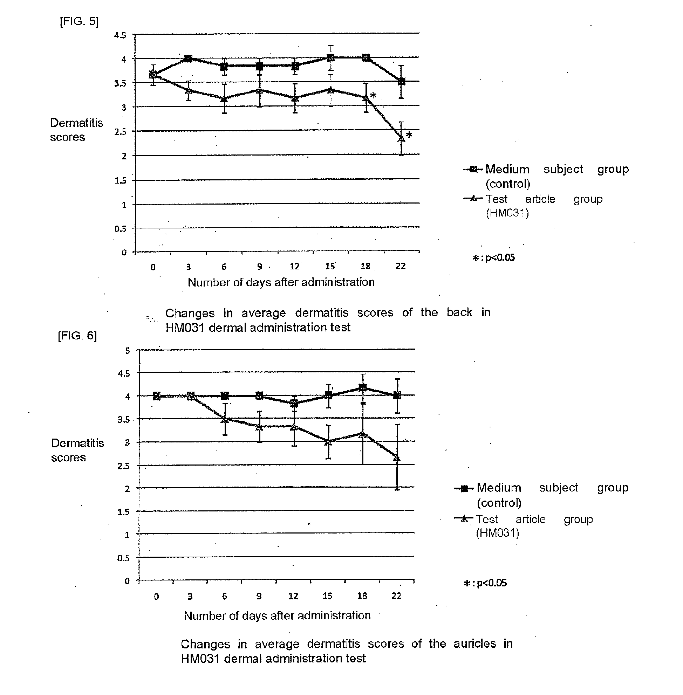

Changes in dermatitis scores of the whole area (back and auricles) are given in Table 5 and FIG. 3, those of the back are given in Table 6 and FIG. 34, and those of the auricles are given in Table 7 and FIG. 5.

**[Table 5]**

| Changes in average dermatitis scores of the whole area in HM031 dermal administration test | | | | | | | | |
|---|---|---|---|---|---|---|---|---|
| Test article | Number of days after administration | | | | | | | |
| | Average dermatitis scores (back and auricles) ^{a)} | | | | | | | |
| | 0 | 3 | 6 | 9 | 12 | 15 | 18 | 22 |
| Medium subject group (control) | 7.7±0.21 | 8.0±0 | 78±0.17 | 7.8±0.17 | 7.7±0.21 | 8.0±0.37 | 8.2±0.31 | 7.5±0.62 |
| Test article group (HM031) | 7.7±0.21 | 7.3±0.21 | 6.7±0.61 | 6.7±0.67 | 6.5±0.67 | 6.3±0.67 | 6.3±0.92 | 5.0±0.93 |

| | | | | | | | | |
|---|---|---|---|---|---|---|---|---|
| a) n=6 Average value ± standard error | | | | | | | | |

**[Table 6]**

| Changes in average dermatitis scores of the back in HM031 dermal administration test | | | | | | | | |
|---|---|---|---|---|---|---|---|---|
| Test article | Number of days after administration | | | | | | | |
| | Average dermatitis scores (back) ^{a)} | | | | | | | |
| | 0 | 3 | 6 | 9 | 12 | 15 | 18 | 22 |
| Medium subject group (control) | 3.7±0.21 | 4.0±0 | 3.8±0.17 | 3.8±0.17 | 3.8±0.17 | 4.0±0.26 | 4.0±0 | 3.5±0.34 |
| Test article (HM031) | group 3.7±0.21 | 3.3±0.21 | 3.2±0.31 | 3.3±0.33 | 3.2±0.31 | 3.3±0.33 | 32±0.31 | 2.3±0.33 |

| | | | | | | | | |
|---|---|---|---|---|---|---|---|---|
| a) n=6 Average value ± standard error | | | | | | | | |

**[Table 7]**

| Changes in average dermatitis scores of the auricles in HM031 dermal administration test | | | | | | | | |
|---|---|---|---|---|---|---|---|---|
| Test article | Number of days after administration | | | | | | | |
| | Average dermatitis scores (auricles) ^{a)} | | | | | | | |
| | 0 | 3 | 6 | 9 | 12 | 15 | 18 | 22 |
| Medium subject group (control) | 4.0±0 | 4.0±0 | 4.0±0 | 4.0±0 | 3.8±0.17 | 4.0±0.26 | 4.2±0.31 | 4.0±0.37 |
| Test article group (HM031) | 4.0±0 | 4.0±0 | 3.5±0.34 | 3.3±0.33 | 3.3±0.42 | 3.0±0.37 | 3.2±0.05 | 2.7±0.71 |

| | | | | | | | | |
|---|---|---|---|---|---|---|---|---|
| a) n=6 Average value ± standard error | | | | | | | | |

### 4. Observation of test results

As a result of checking changes in the scores of dermatitis severity due to consecutive dermal administration in Working Example 11, improvement tendencies of the dermatitis is observed from the third day in the whole area (back and auricles) and the back, and from the sixth day in the auricles.

Moreover, according to the Mann-Whitney's U test with significance level of 5%, significant difference from the control group is seen on the twenty-second day in the whole area (back and auricles), and on the eighteenth day and on the twenty-second day in the back.

The Biostir AD model is a model in which lesions are seen in the mouse back and auricles. In order to diagnose in a similar way to human skin, it is better to emphasize healing effect of skin lesions on the mouse back rather than in the soft auricles.

In this test, the fact that a significant dermatitis improvement effect on the mouse back is confirmed in Working Example 11 suggests that the significant dermatitis improvement effect in Working Example 11 is promising for humans.

In addition, changes in dermatitis scores due to dermal administration of Locoid ointment (registered trademark, manufactured by Torii Pharmaceutical Co., Ltd., steroid rank group IV, mild) are shown as control samples in FIG. 6.

In the Locoid ointment administration test, while significant difference in the Mann-Whitney's U test is not confirmed until the fifth day, dermatitis improvement tendencies are confirmed from around the third day.

From these results, while it is difficult to compare them equally since there is difference in number of observation days in this test and the Locoid ointment test, Working Example 11, in the dermal administration test using Biostir AD-induced atopic dermatitis model mice, suggests a possibility of showing approximately the same or higher level of dermatitis improvement effect as with the Locoid ointment.

An additional example of a representative embodiment is given forthwith.

While the above application example of an application-type external medicine in which a first layer containing a metal or metal hydride and a second layer containing a citric acid solution (and platinum microparticles as needed) contrapose each other via a screen board such as a plastic plate, a fabric product such as a pillow or cushion may be designed using the same structure. In other words, a fabric product, in which by pulling out the screen board at bedtime or the like, the first layer and the second layer make contact, generating active hydrogen or activating hydrogen molecules, as well as supplying hydrogen molecules or active hydrogen to area of contact such as the head and backside, may be designed. Such fabric product may be used for prevention and/ or treatment of diseases involving cognitive impairment, cerebral infarction, or oxidant stress such as bed sores, for example. Alternatively, a structure, in which a powder containing citric acid and a metal or metal hydride is simply mixed with an aprotic solvent or nonpolar solvent as needed, and hydrogen molecules or active hydrogen is generated in an area of contact such as the head via moisture such as sweat exuding from the said area, may be employed.

According to yet another embodiment, the 'occlusive dressing technique' used when applying to skin a steroid chemical in treatment for atopic dermatitis and similar diseases may be used in combination. In other words, when applying the external medicine, since hydrogen molecules or active hydrogen is made to penetrate through the skin or a mucous membrane, in addition to application of the external medicine, a wrap or sheet made of a material such as polyethylene or polyvinylidene chloride may cover the area. At this time, for also further heightening sealing performance of hydrogen, material of the wrap or sheet is preferably made from a gas impermeable material (e.g., aluminum) for hydrogen gas or the like.

According to yet another embodiment, well-known liposome may be used as the external medicine. Liposome is artificial cytoid microparticles constituted by phospholipid, which constitutes cell membranes of organisms, and may include water-soluble or lipid-soluble chemicals. Since biocompatibility is high and the chemical may be transferred in the body while being protected from catabolic enzymes, it may be used as a carrier for chemical compounds such as chemical drugs. While the present invention is not limited thereto, liposome, which has a hollow interior and is referred to as hollow liposome or the like, may be used preferably. In other words, a liposome suspension is removed as needed through decompression or similar process, and gas including hydrogen is filled in the head space of a container containing the suspension in a suitable atmospheric pressure environment (e.g., 1 to 10 atm or 1.5 to 10 atm) so as to maintain it for a suitable duration (e.g., 1 to 30 minutes). As a result, hydrogen penetrates into the suspension within the head space, and permeates into the interior via a film constituting the liposome. At this time, ultrasonic waves or the like may be irradiated (e.g., ultrasonic waves of 20 to 50 kHz for 1 to 5 minutes) so as to increase the permeation efficiency. Afterward, by decompressing over a suitable period of time (e.g., 1 to 10 minutes), area of the hydrogen within the liposome swells and is retained stably in the film. The hydrogen-containing liposome suspension prepared in this manner may be used as the external medicine according to the present invention. In other words, hydrogen may be generated in the skin or a mucous membrane by either mixing the hydrogen-contained liposome suspension with the second agent, which contains a substance (e.g., alcohol such as ethanol or a surface acting agent) having a function of breaking down the film constituting the liposome, or by breaking down the film with physical force such as ultrasound waves. Moreover, in light of compatibility between the liposome and skin, application directly on the skin or the mucous membrane without using such second agent allows the free radical or active oxygen species generated in the skin or the mucous membrane, which may be generated due to ultraviolet rays or the like, to break down the film constituting the liposome. In other words, hydrogen is released (generated) from the liposome due to breaking down of the film, and the released hydrogen molecules are then oxidized by the free radical or active oxygen species and are thus activated. Therefore, an effective external medicine, which takes effect in such a way that being attacked (oxidized) leads directly to counterattack (antioxidation) may be structured.

Additional working examples are described next.

### [Reducing power evaluation test through coloration change of methylene blue]

### 1. Test procedure

This test is conducted according to the aforementioned test procedure.

### 2. Presentation of working examples, comparative examples, and reference examples

### [Working Example 12]

50 mg of atomized magnesium powder (-40 mesh) manufactured by Tangshan Weihao Magnesium Powder Co., Ltd. is used as a first agent, and 950 mg of a solution resulting from diluting two-hundredfold purified water, which is Fujisawa City tap water processed through an ion-exchange column manufactured by Organo Corporation, with a 4 wt% platinum colloid solution manufactured by Tanaka Kikinzoku Kogyo K.K., and adding citric acid to 20% concentration is used as a second agent.

A mixture of the first agent and the second agent is used as a test article. After adjustment of the test article, 1 mL of each of the methylene blue aqueous solutions is dripped thereupon while visually observing color change of the test article. Quantity of the methylene blue aqueous solutions dripped until color change is no longer confirmed is 7 mL for a solution of 2.5 g/L concentration, and 2 mL for that of 1 g/L concentration, and total reduced methylene blue is 61.0 µmol.

### [Working Example 13]

50 mg of magnesium hydride manufactured by Alfa Aesar (ALF) is used as a first agent, and 950 mg of solution resulting from diluting two-hundredfold purified water, which is Fujisawa City tap water processed through an ion-exchange column manufactured by Organo Corporation, with a 4 wt% platinum colloid solution manufactured by Tanaka Kikinzoku Kogyo K.K. is used as a second agent.

A mixture of the first agent and the second agent is used as a test article. After adjustment of the test article, 1 mL of each of the methylene blue aqueous solutions is dripped thereupon while visually observing color change of the test article. Quantity of the methylene blue aqueous solutions dripped until color change is no longer confirmed is 4 mL for a solution of 2.5 g/L concentration, and total reduced methylene blue is 31.3 µmol.

### [Antioxidative activity evaluation test 2 through coloration change of DPPH radicals]

### 1. Test procedure

As a basic test procedure, 1 g of each test article is prepared, operation for generating hydrogen is performed as needed, and a DPPH radical solution adjusted to a constant concentration is dripped thereupon. If these test articles have antioxidative activity, the dripped purple-colored DPPH radical solution will be reduced and turn amber; however, if quantity of the DPPH radical solution poured in is gradually increased, and the antioxidant within the test articles is completely consumed, coloration change of the purple color of the DPPH radical solution to amber cannot be observed. Antioxidative activity that each of the test articles has is evaluated based on the total dripped quantity of the DPPH radical solution until this time in the respective articles.

### A specific testing method is described next.

As DPPH radical solutions to be dripped, prepare a 0.1 g/L (volume molarity: 0.253 mM) one, a 1 g/L (volume molarity: 2.53 mM) one, and a 5 g/L (volume molarity: 12.65 mM) one. Here, three types of DPPH radical solutions differing in concentration are prepared because since there is difference in antioxidative activity among the test articles, higher test accuracy may be expected if three types of DPPH radical solutions differing in concentration are properly used according to antioxidative activity provided to the respective test articles.

Next, as to the environment when DPPH is actually dripped, since hydrogen activated on the surface of precious metal microparticles or catalyst reduces the DPPH, and at the same time, the oxygen in the solution or the air reacts therewith and is consumed, testing should be conducted in an enclosed environment essentially, using test articles expelled of any existing air.

However, this test is in a different situation. Supposing that an enclosed environment is created by plugging test tubes with rubber plugs, and generation and reaction of hydrogen and dripping of the DPPH is performed in that environment, in the case of making, for example, calcium and water react, hydrogen is generated vigorously, the rubber plugs will be blown off, and the enclosed environment cannot be maintained. This fact is not limited to only calcium, and the same matter can be said about other several test articles to which this test is conducted. Moreover, empirically, as a result of considering that impact (approximately several ppm) of oxygen in the reagent and impact due to reaction with the oxygen in the air is small, and is not a big problem upon comparison among the test articles, it has been decided to conduct this test in air without making a point to create an oxygen-free environment.

20 mL test tubes are used as the containers. Place 1 g of the test article in each of the test tubes, and drip 1 mL of the respective DPPH radical solutions therein using a pipet. Shake and stir the test tubes each time 1 mL is dripped in while visually observing for any color reaction. Note that color reaction of the DDPH radical is irreversible, and DPPH that has been reduced once and has turned to an amber color will not return to a purple color even if it is oxidized in air. However, considering that hydrogen is consumed by the oxygen in the air, dripping of the DPPH radical solutions must be carried out quickly.

### 2. Presentation of working examples, comparative examples, and reference examples

### [Working Example 14]

50 mg of magnesium powder ((-100 + 200 mesh, 99.6%) manufactured by Alfa Aesar (ALF) is used as a first agent, and 950 mg of solution resulting from diluting two-hundredfold purified water, which is Fujisawa City tap water processed through an ion-exchange column manufactured by Organo Corporation, with a 4 wt% platinum colloid solution manufactured by Tanaka Kikinzoku Kogyo K.K., and adding citric acid to 20% concentration is used as a second agent.

A mixture of the first agent and the second agent is used as a test article. After adjustment of the test article, 1 mL of each of the DPPH radical solutions is dripped thereupon while visually observing color change of the test article. Quantity of the DPPH radical solutions dripped until color change is no longer confirmed is 3 mL for a solution of 5 g/L concentration, and 1 mL for that of 1 g/L concentration, and total reduced DPPH radicals is 40.48 µmol.

### [Working Example 15]

50 mg of zinc powder manufactured by Wako Pure Chemical Industries, Ltd.
is used as a first agent, and 950 mg of solution resulting from diluting two-hundredfold purified water, which is Fujisawa City tap water processed through an ion-exchange column manufactured by Organo Corporation, with a 4 wt% platinum colloid solution manufactured by Tanaka Kikinzoku Kogyo K.K., and adding citric acid to 20% concentration is used as a second agent.

A mixture of the first agent and the second agent is used as a test article. After adjustment of the test article, 1 mL of each of the DPPH radical solutions is dripped thereupon while visually observing color change of the test article. Quantity of the DPPH radical solutions dripped until color change is no longer confirmed is 4 mL for a solution of 5 g/L concentration, and 1 mL for that of 1 g/L concentration, and total reduced DPPH radicals is 58.19 µmol.

### [Working Example 16]

50 mg of calcium granules manufactured by Wako Pure Chemical Industries, Ltd. is used as a first agent, and 950 mg of solution resulting from diluting two-hundredfold purified water, which is Fujisawa City tap water processed through an ion-exchange column manufactured by Organo Corporation, with a 4 wt% platinum colloid solution manufactured by Tanaka Kikinzoku Kogyo K.K. is used as a second agent.

A mixture of the first agent and the second agent is used as a test article. After adjustment of the test article, 1 mL of each of the DPPH radical solutions is dripped thereupon while visually observing color change of the test article. Quantity of the DPPH radical solutions dripped until color change is no longer confirmed is 3 mL for a solution of 5 g/L concentration, and 1 mL for that of 1 g/L concentration, and total reduced DPPH radicals is 40.48 µmol.

### [Working Example 17]

50 mg of sodium borohydride manufactured by Merck & Co., Inc. is used as a first agent, and 950 mg of solution resulting from diluting two-hundredfold purified water, which is Fujisawa City tap water processed through an ion-exchange column manufactured by Organo Corporation, with a 4 wt% platinum colloid solution manufactured by Tanaka Kikinzoku Kogyo K.K. is used as a second agent.

A mixture of the first agent and the second agent is used as a test article. After adjustment of the test article, 1 mL of each of the DPPH radical solutions is dripped thereupon while visually observing color change of the test article. Quantity of the DPPH radical solutions dripped until color change is no longer confirmed is 4 mL for a solution of 5 g/L concentration, and 1 mL for that of 1 g/L concentration, and total reduced DPPH radicals is 53.13 µmol.

### [Working Example 18]

50 mg of magnesium powder ((-100 + 200 mesh, 99.6%) manufactured by Alfa Aesar (ALF) is used as a first agent, and 950 mg of solution resulting from diluting two-hundredfold purified water, which is Fujisawa City tap water processed through an ion-exchange column manufactured by Organo Corporation, with a 4 wt% palladium colloid solution manufactured by Tanaka Kikinzoku Kogyo K.K., and adding citric acid to 20% concentration is used as a second agent.

A mixture of the first agent and the second agent is used as a test article. After adjustment of the test article, 1 mL of each of the DPPH radical solutions is dripped thereupon while visually observing color change of the test article. Quantity of the DPPH radical solutions dripped until color change is no longer confirmed is 2 mL for a solution of 5 g/L concentration, and 1 mL for that of 1 g/L concentration, and total reduced DPPH radicals is 27.83 µmol.

### [Working Example 19]

50 mg of magnesium powder ((-100 + 200 mesh, 99.6%) manufactured by Alfa Aesar (ALF) is used as a first agent, and 950 mg of solution resulting from adding citric acid to purified water, which is Fujisawa City tap water processed through an ion-exchange column manufactured by Organo Corporation, to 20% concentration is used as a second agent.

A mixture of the first agent and the second agent is used as a test article. After adjustment of the test article, 1 mL of each of the DPPH radical solutions is dripped thereupon while visually observing color change of the test article. Quantity of the DPPH radical solutions dripped until color change is no longer confirmed is 5 mL for a solution of 0.1 mg/L concentration, and total reduced DPPH radicals is 1.27 µmol.

### [Comparative Example 6]

1 mL of a solution resulting from adding a 4 wt% platinum colloid solution manufactured by Tanaka Kikinzoku Kogyo K.K. to saturated hydrogen water, which results from bubbling hydrogen gas for 90 minutes into purified water or Fujisawa City tap water processed through an ion-exchange column manufactured by Organo Corporation to a 200 mg/L platinum colloid concentration, is used as a test article. After adjustment of the test article, 1 mL of each of the DPPH radical solutions is dripped thereupon while visually observing color change of the test article. Quantity of the DPPH radical solutions dripped until color change is no longer confirmed is 1 mL for a solution of 0.1 mg/L concentration, and total reduced DPPH radicals is 0.25 µmol.

### [Comparative Example 7]

1 mL of a solution resulting from adding Japanese Pharmacopoeia ascorbic acid manufactured by Kokando Pharmaceutical Co., Ltd. to purified water or Fujisawa City tap water processed through an ion-exchange column manufactured by Organo Corporation to a 1000 mg/L concentration, is used as a test article. 1 mL of each of the DPPH radical solutions is dripped thereupon while visually observing color change of the test article. Quantity of the DPPH radical solutions dripped until color change is no longer confirmed is 1 mL for a solution of 5 g/L concentration, and 1 mL for that of 1 g/L concentration, and total reduced DPPH radicals is 15.18 µmol.

### [Reference Example 4]

1 mL of saturated hydrogen water, which results from bubbling hydrogen gas for 90 minutes into purified water or Fujisawa City tap water processed through an ion-exchange column manufactured by Organo Corporation, is used as a test article. After adjustment of the test article, 1 mL of each of the DPPH radical solutions is dripped thereupon while visually observing color change of the test article. 1 mL of the DPPH radical solution with the weakest concentration of 0.1 mg/L could not be turned transparent.

### [Reference Example 5]

1 mL of a solution resulting from diluting two-hundredfold purified water, which is Fujisawa City tap water processed through an ion-exchange column manufactured by Organo Corporation, with a 4 wt% platinum colloid solution manufactured by Tanaka Kikinzoku Kogyo K.K., and adding citric acid to 20% concentration is used as a test article. After adjustment of the test article, 1 mL of each of the DPPH radical solutions is dripped thereupon while visually observing color change of the test article. 1 mL of the DPPH radical solution with the weakest concentration of 0.1 g/L could not be turned transparent.

### [Reference Example 6]

1 mL of a solution resulting from diluting two-hundredfold purified water, which is Fujisawa City tap water processed through an ion-exchange column manufactured by Organo Corporation, with a 4 wt% platinum colloid solution manufactured by Tanaka Kikinzoku Kogyo K.K., is used as a test article. After adjustment of the test article, 1 mL of each of the DPPH radical solutions is dripped thereupon while visually observing color change of the test article. 1 mL of the DPPH radical solution with the weakest concentration of 0.1 mg/L could not be turned transparent.

### [Hydroxy radical (OH radical) elimination measurement using the electronic spin resonance method (ESR)]

Next, elimination of a hydroxy radical due to active hydrogen is measured using the electronic spin resonance method.

### 1. OH radical generation system

An OH radical generation system includes the Fenton reaction system and a hydrogen peroxide photolysis system. With the former system, an OH radical and Fe(III) are generated from a reaction between Fe(II) and hydrogen peroxide. When a strong reducing agent coexists in the measurement system, other than elimination of the OH radical, there is a risk of re-reducing of Fe(III), and causing generation of an OH radical through reaction with the remaining hydrogen peroxide.

### Therefore, photolysis of hydrogen peroxide is used in this evaluation.

### 2. Testing

### 2-1 Reagent

A stock solution 30% of hydrogen peroxide (manufactured by Wako Pure Chemical Industries, Ltd.) is diluted with purified water to prepare 75 mM aqueous solution. 5,5-Dimethyl-1-pyrroline N-Oxide (DMPO manufactured by Labotec Co., Ltd.) is used as a spin trapping agent to prepare a 1 M concentration aqueous solution using purified water. A solution resulting from adding citric acid to purified water or Fujisawa City tap water processed through an ion-exchange column manufactured by Organo Corporation, to 10 wt% concentration is used as the citric acid aqueous solution. Magnesium powder (-100 + 200 mesh, 99.6%) manufactured by Alfa Aesar (ALF) is used as the magnesium (Mg).

### 2-2 Apparatus

A JES-FR30 free radical monitor manufactured by JEOL Ltd. is used for ESR spectrum measurement. Conditions for measurement are as follows. Magnetic field: 336 ± 5 mT, microwave output: 4 mW, magnetic field modulation: 0.1 mT, amplification rate: appropriately adjusted. According to an internal standard using manganese markers, the third and fourth signals from the low magnetic field side are adjusted so as to be recorded within the range of the magnetic field. In order to irradiate ultraviolet rays and visible light, a xenon lamp (Luminar Ace, LA-100UV manufactured by Hayashi Watch-works Co., Ltd.) is used to irradiate an illumination intensity of 10000 1x.

### 2-3 Measurement procedure

A method of generating an OH radical to produce an adduct such as DMPO-OH is used according to the following procedure. 160 µL citric acid, 20 µL DMPO aqueous solution, and 20 µL hydrogen peroxide solution are introduced in a glass test tube and exposed to light for 5 seconds using the xenon lamp. Immediately, the solution is extracted to a hematocrit tube, and ESR spectrum measurement is started 30 seconds later. As for the Mg series that makes foam, 5 mg of a metal powder corresponding thereto is put in a glass test tube, the aforementioned, premixed 160 µL citric acid, 20 µL DMPO aqueous solution, and 20 µL hydrogen peroxide solution (total quantity of 200 µL) are added therein, and it is immediately exposed to light for 5 seconds. Once the foaming nearly stops, the solution is transferred to a hematocrit tube, and ESR spectrum is measured 30 seconds later.

### 3. Results and observation

### 3-1 OH radical generation system

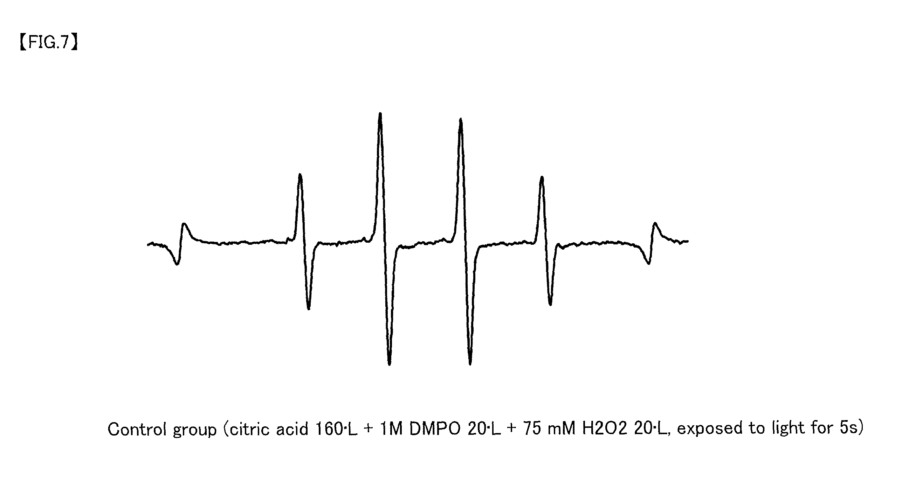

As shown in FIG. 7, under conditions of solution concentrations of 160 µL citric acid, 20 µL DMPO aqueous solution, and 20 µL hydrogen peroxide solution, illuminating radiation intensity, and irradiation time , ESR signal strength of DMPO-OH with relatively good reproducibility is obtained. Concentration thereof is estimated to be approximately 0.1 mM, where the g value is 2.0055.

### 3-2 Mg series

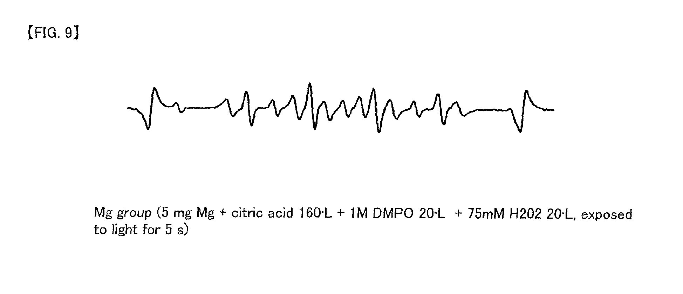

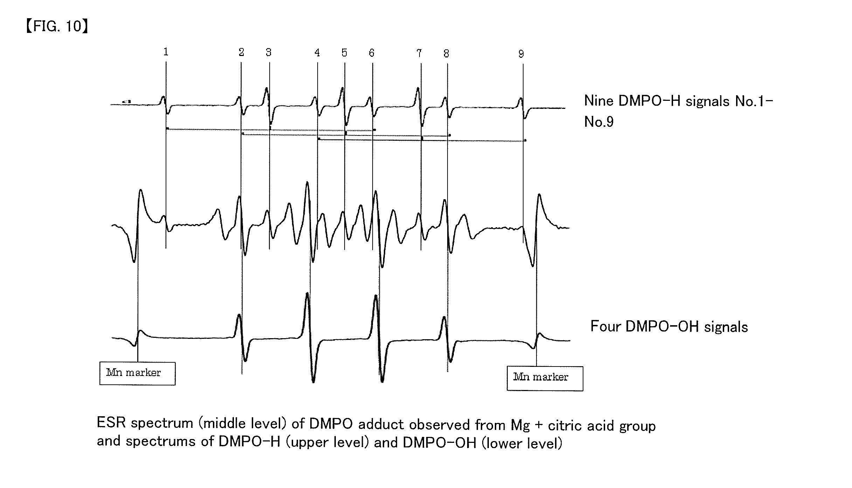

As shown in FIG. 9, an ESR spectrum of the DMPO adduct is obtained. This indicates that a DMPO-OH signal and a DMPO-H signal derived from active hydrogen (hydrogen in a nascent state) in the initial stage of hydrogen gas generation are generated (See FIG. 10.) A total of nine DMPO-H signals are observed, and the one appearing in the weakest magnetic field is to the immediate right of the manganese marker. While analysis is difficult due to noise, DMPO-OH and DMPO-H are surely observed.

Moreover, the signal strength of DMPO-OH is clearly diminishing compared to the control system of FIG. 7 (comparison with signal on weakest magnetic field side). This diminished amount is said to have eliminated OH radicals.

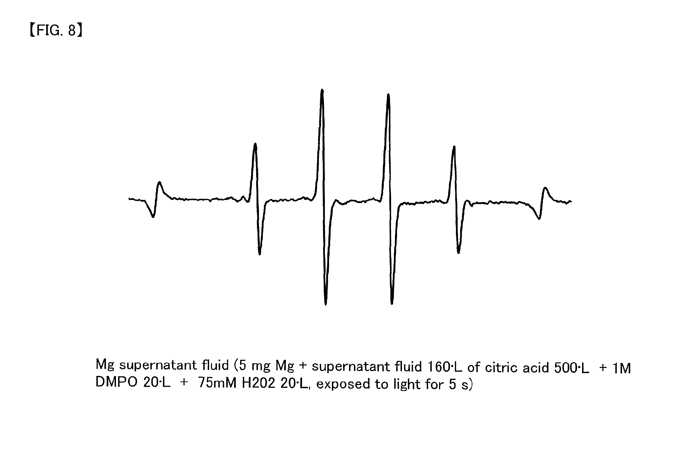

Note that FIG. 8 is an ESR spectrum of an observed, so-called supernatant fluid that is distant from the foaming site other than a foaming site in an initial stage of generating hydrogen gas. The signal strength of DMPO-OH is not diminishing compared to the control system of FIG. 7, and the DMPO-H signal is not observed.

It can be understood from this finding as well that with the external medicine of the present invention, it is important that instead of bringing the hydrogen molecules (or solution containing hydrogen molecules) generated by the external medicine into contact with skin or a mucous membrane, either the external medicine itself is kept in the skin or mucous membrane so as to allow generation of hydrogen (active hydrogen) in the target region, or the external medicine itself is brought into contact with the target region during any of the processes in which hydrogen (active hydrogen) is generated. Even if the external medicine is brought into contact with skin in the stage where generation is finished, (even if there is a possibility of hydrogen molecules being in contact with the target region,) good results from hydrogen generated (foaming) in a nascent state cannot be expected.

Note that while, as mentioned before, the concept of active hydrogen in this application does not only include hydrogen molecules activated by a catalyst (atomic hydrogen or hydride ion), but also includes an aspect of the hydrogen molecules being forcibly oxidized (take out the hydrogen) by a strong oxidative radical, needless to say, it is a concept further including atomic hydrogen, hydride ions, or electrons themselves at the instant that hydrogen is generated by an active hydrogen generating agent such as hydrogen in a 'nascent state' as shown by this test, namely a metal or metal hydride.

### [Preparation method of external medicine for treatment or prevention: addition of composition examples]

A composition example of a case where the present invention, while not limited hereto, is structured as an external medicine not containing a catalyst is given forthwith. Note that even in this composition example and the aforementioned composition example using the magnesium powder MG100, in light of usage as an external medicine and formulation balance (push out in even amounts from a dual dispenser container, or pulverize a capsule first agent in a solution state second agent, or the like), the first agent and the second agent are, while not limited thereto, in a ratio where when the first agent is set to 1, the second agent has a weight percent of 0.001 to 10000, preferably 0.01 to 1000, more preferably 0.1 to 100, and most preferably 0.5 to 50.

### First agent:

2.0 wt% atomized magnesium powder (-400 mesh) (manufactured by Tanshan Weihao Magnesium Powder Co., Ltd.)
2.0 wt% hydroxypropylcellulose (Nippon Soda Co., Ltd.)
10 wt% glycerin (TOHO Medicine Manufacturing Co. Ltd.)
0.05 wt% Calfa (grapefruit seed extract, preservative) (Calfa Chemical Co., Ltd.)
2.0 % AEROSIL 200 (Nippon Aerosil Co., Ltd.)
42.0 wt% dimethylsulfoxide (Wako Pure Chemical Industries, Ltd.)
41.95 wt% purified water
or,
First agent:
1.0 wt% magnesium hydride (Alfa Aesar)
2.0 wt% Metolose (methylcellulose) SNB-30T (Shin-Etsu Chemical Co., Ltd.)
10 wt% glycerin (TOHO Medicine Manufacturing Co. Ltd.)
1.0 % AEROSIL 200 (Nippon Aerosil Co., Ltd., antisettling agent)
0.05 wt% Calfa (grapefruit seed extract, preservative) (Calfa Chemical Co., Ltd.)
83.95 wt% dimethylsulfoxide (Wako Pure Chemical Industries, Ltd., aprotic solvent) Second agent:
   10.0 wt% citric acid (Wako Pure Chemical Industries, Ltd.)
   0.5 wt% hydroxypropylcellulose (Nippon Soda Co., Ltd.)
   5 wt% glycerin (TOHO Medicine Manufacturing Co. Ltd.)
   0.05 wt% Calfa (grapefruit seed extract, preservative) (Calfa Chemical Co., Ltd.) 84.45 wt% purified water

When structuring the external medicine with viscosity as in this composition example, in light of effect, particle dispersibility, and the like, content of the active hydrogen generating agent such as a metal or metal hydride within the first agent should be 0.001 to 100 wt%, preferably 0.01 to 50 wt%, more preferably 0.10 to 25 wt%, most preferably 0.25 to 10 wt%, but is not limited thereto. Moreover, when including acid in the second agent, in light of reactivity of the metal or metal hydride to be used, proton donating capability of the acid, and the like, content thereof should be 0.01 to 99 wt%, preferably 0.25 to 90 wt%, more preferably 0.5 to 60 wt%, most preferably 1 to 30 wt%, but is not limited thereto. Furthermore, while it is not included in this composition example, when including precious metal microparticles such as platinum as a catalyst within the second agent as in the aforementioned composition example, in light of reaction rate, material cost, and the like, content thereof should be 0.005 to 500000 mg/L, preferably 0.05 to 50000 mg/L, more preferably 0.5 to 5000 mg/L, most preferably 5 to 500 mg/L, but is not limited thereto. Yet further, when including an antisettling agent in the first agent, in light of specific gravity, quantity consumed, and the like, of the metal or metal hydride to be used, content thereof should be 0.01 to 50 wt%, preferably 0.25 to 25 wt%, more preferably 0.5 to 12.5 wt%, most preferably 0.75 to 6.25 wt%, but is not limited thereto. Yet even further, when including a protic solvent or nonpolar solvent in the first agent, in light of quantity consumed of the metal or metal hydride, reactivity with the protic solvent, and the like, content thereof should be 1 wt% or greater, preferably 25 wt% or greater, more preferably 50 wt% or greater, most preferably 75 wt% or greater, but is not limited thereto. Yet even further, the viscosity (measured at an agent temperature of 20 degrees Celsius using the TVB-10 viscometer manufactured by Toki Sangyo Co., Ltd.) of the first agent should be 1 to 64000000 mPA•s, preferably 10 to 6400000 mPA•s, more preferably 100 to 640000 mPA•s, most preferably 1000 to 64000 mPA•s in light of agent form, thixotropy, and the like, but is not limited thereto. In light of agent form, thixotropy, and the like, the viscosity (measured at an agent temperature of 20 degrees Celsius using the TVB-10 viscometer manufactured by Toki Sangyo Co., Ltd.) of the second agent should be 1 to 64000000 mPA•s, preferably 2 to 6400000 mPA•s, more preferably 3 to 640000 mPA•s, most preferably 4 to 64000 mPA•s, but is not limited thereto. Yet even further, the viscosity (composite resulting from mixing the first agent and the second agent and leaving it for at least 72 hours is measured at an agent temperature of 20 degrees Celsius using the TVB-10 viscometer manufactured by Toki Sangyo Co., Ltd.) of the composite of the first agent and the second agent should be 1.25 to 256000 mPA•s, preferably 2.5 to 192000 mPA•s, more preferably 5 to 256000 mPA•s, most preferably 10 to 64000 mPA•s in light of retaining ability in skin or a mucous membrane and feeling of use, but is not limited thereto.

[Temporal deterioration inhibition test for metal or metal hydride due to mixing of aprotic solvent or nonpolar solvent in the solvent of the first agent]
If a chemical compound having remarkable reactivity such as calcium hydride is removed, the metal or metal hydride reacting with acid and generating hydrogen does not easily react with a protic solvent having a pH in the neutral to alkali region such as tap water. Meanwhile, however, since hydrogen is generated albeit a little bit if it is dispersed in water, for example, even with such a metal or metal hydride, the metal or metal hydride, which is an action component within the first agent, is lost with time. Therefore, while formation of a passive film on the particle surfaces as described above, and replacement of a part of an aprotic solvent or nonpolar solvent for the protic solvent is important, in this case, comparison of hydrogen concentration in container head spaces when water or protic solvent in the first agent is replaced in stages by dimethyl sulfoxide (DMSO) or aprotic solvent shows how the aprotic solvent suppresses temporal attenuation of the metal or metal hydride. Note that since the objective of this test is as described above, as an additional remark, quantity of hydrogen stated in this test is not correlated much with quantity of hydrogen actually generated by the metal or metal hydride. The fact is that while this test has been conducted under handmade, loose circumstances as described below, if it is an objective to accurately measure the quantity of hydrogen in the container head space, it goes without saying that the water replacement method described below should be employed.

### 1. Test procedure

Each test article is placed in a 420 cc glass container, and the opening is covered with a polyvinylidene chloride wrap. It is then further covered with aluminum foil and tightly fixed with a rubber band around the container from on top of the aluminum foil, thereby creating a semi-sealed state. After about 17 hours, a hole is carefully formed in the wrap and the foil, a hose is inserted therein, and concentration of the hydrogen accumulated in the head space of the glass container is measured using a dissolved hydrogen meter 'DHDI-1' manufactured by DKK-TOA Corporation. Essentially, the dissolved hydrogen meter is a device for measuring dissolved hydrogen in a liquid phase; however, this method is adopted in order to avoid damaging apparatus due to sucking up of magnesium hydride that disperses in the test articles, and taking into consideration that the objective of this test is not to accurately measure hydrogen quantity.

### 2. Test articles

Test article 1: 17.50 g DMSO (manufactured by Wako Pure Chemical Industries, Ltd.), 0.25 g magnesium hydride (manufactured by Alfa Aesar), and 7.50 g purified water, which is Fujisawa City tap water processed through an ion-exchange column manufactured by Organo Corporation.

Test article 2: 12.50 g DMSO (manufactured by Wako Pure Chemical Industries, Ltd.), 0.25 g magnesium hydride (manufactured by Alfa Aesar), and 12.50 g purified water, which is Fujisawa City tap water processed through an ion-exchange column manufactured by Organo Corporation.

Test article 3: 7.50 g DMSO (manufactured by Wako Pure Chemical Industries, Ltd.), 0.25 g magnesium hydride (manufactured by Alfa Aesar), and 17.50 g purified water, which is Fujisawa City tap water processed through an ion-exchange column manufactured by Organo Corporation.

Test article 4: 0.00 g DMSO (manufactured by Wako Pure Chemical Industries, Ltd.), 0.25 g magnesium hydride (manufactured by Alfa Aesar), and 25.00 g purified water, which is Fujisawa City tap water processed through an ion-exchange column manufactured by Organo Corporation.

### 3. Results and observation

The hydrogen concentration in the head space of the glass container into which test article 1 is placed is 0.002 ppm. The hydrogen concentration in the head space of the glass container into which test article 2 is placed is 0.007 ppm. The hydrogen concentration in the head space of the glass container into which test article 3 is placed is 0.017 ppm. The hydrogen concentration in the head space of the glass container into which test article 4 is placed is 0.058 ppm.

As indicated by the test results, the more of the aprotic solvent or nonpolar solvent is included as a solvent (dispersion medium) in the agent (first agent) containing the metal or metal hydride, the less hydrogen is generated. In other words, when the present invention has a configuration that generates active hydrogen by bringing multiple agents into contact, since it goes without saying that maintaining the agent containing the metal or metal hydride in a stable state within the solvent (dispersion medium) is preferred, replacing the protic solvent of the first agent with an appropriate aprotic solvent or nonpolar solvent in light of usage as an external medicine, cost, and the like is preferred.
When the protic solvent in the first agent is assumed to be 1, weight percent of the aprotic solvent or nonpolar solvent to the protic solvent is preferably 0 or greater, 1 or greater, 2 or greater, 3 or greater, 4 or greater, 5 or greater, 6 or greater, 7 or greater, 8 or greater, 9 or greater, and 10 or greater, and all or almost all of the solvent (dispersion medium) within the first agent may be the aprotic solvent or nonpolar solvent.

[Measurement of quantity of generated hydrogen from metal magnesium and magnesium hydride using water replacement method (Kanagawa Industrial Technology Center)]

### 1. Test procedure

A flask with appropriate capacity is installed, and a glass tube is extended until the measuring cylinder, which is installed inverted in a beaker with water at a side of the flask. Once gas that has emerged at the beginning is discarded, the respective test articles are placed in the flask in order of metal and solution. The gas volume accumulated in the head space of the measuring cylinder is measured using calibrations of the measuring cylinder.

### 2. Test articles

Test article 1: 0.5 g metal magnesium (manufactured by Alfa Aesar) (particle diameter 150 µm or less), and 30 g of purified water, which is Fujisawa City tap water processed through an ion-exchange column manufactured by Organo Corporation. Test article 2: 0.5 g metal magnesium (manufactured by Alfa Aesar) (particle diameter 150 µm or less), and 30 g of solution, which results from dissolving citric acid (manufactured by Wako Pure Chemical Industries, Ltd.) in Fujisawa City tap water processed through an ion-exchange column manufactured by Organo Corporation to 5wt%.

Test article 3: 0.5 g metal magnesium (manufactured by Alfa Aesar) (particle diameter 150 µm or less), and 30 g of solution, which results from dissolving citric acid (manufactured by Wako Pure Chemical Industries, Ltd.) in Fujisawa City tap water processed through an ion-exchange column manufactured by Organo Corporation to 10wt%.

Test article 4: 0.5 g magnesium hydride (manufactured by Alfa Aesar), and 30 g of purified water, which is Fujisawa City tap water processed through an ion-exchange column manufactured by Organo Corporation.

Test article 5: 0.5 g metal magnesium (manufactured by Alfa Aesar), and 30 g of solution, which results from dissolving citric acid (manufactured by Wako Pure Chemical Industries, Ltd.) in Fujisawa City tap water processed through an ion-exchange column manufactured by Organo Corporation to 10wt%.

### 3. Results and observation

Test article 1 generates 5 mL of hydrogen at a peak after about 10 minutes, test article 2 generates 280 mL of hydrogen at a peak after about 10 minutes, test article 3 generates 485 mL of hydrogen at a peak after about 2.5 minutes, test article 4 generates 0 mL of hydrogen, and test article 5 generates 840 mL of hydrogen at a peak after about 12 minutes. As described above, while quantity of hydrogen generated from 10g of metal magnesium is calculated to be 830 mg, if calculated in the same manner, for each 10g of metal magnesium, test article 1 generates 8.9 mg of hydrogen, test article 2 generates 500 mg, and test article 3 generates 866 mg. In other words, it is estimated that test article 3 or 30 g of a 10 wt% citric acid solution is appropriate as enough solution to completely dissolve 0.5 g of metal magnesium. Through further calculation, it is estimated that enough solution to completely dissolve 1 g of metal magnesium is 60 g of a 10 wt% citric acid solution or 30 g of a 20 wt% citric acid solution. Approximate optimum quantity and concentration balance of metal and acid may be calculated in the same manner with kind of metal and acid used, metal quantity, acid concentration, and acid solution quantity as variables. In other words, a theoretical value of quantity of hydrogen that may develop from an appropriate quantity of the metal or metal hydride is obtained from a chemical reaction formula between the metal or metal hydride and a protic solvent such as water under normal temperature and pressure. In addition, using a hydrogen quantity or volume measuring method such as water replacement method, a function with which a value approaching the theoretical value when the metal is made to react with an appropriate acid such as citric acid can be obtained with quantity of the metal, concentration of the acid, and quantity of solution of the acid as variables. Based on this function, it is possible to appropriately adjust quantity consumed of the metal or metal hydride, concentration of the acid, and quantity of solution of the acid to be used for the external medicine when appropriate values are substituted for two of the variables of quantity of the metal, concentration of the acid, and quantity of solution of the acid, through calculation of the remaining variable, in light of quantity (concentration) of hydrogen to be generated and combination balance of each component. For example, a chemical reaction formula of magnesium hydride and water is:

MgH₂ + 2H₂O → Mg(OH)₂ + 2H₂

Namely, 2 mol (4 g) of hydrogen is theoretically generated for 1 mol (26 g) of magnesium hydride. In other words, 76.923 mg of hydrogen is theoretically generated for 0.5 g of magnesium hydride. As mentioned above, in this test, test article 4 generated 0 mL of hydrogen and test article 5 generated 840 mL of hydrogen. Since the 840 mL of hydrogen generated by test article 5 is 75 mg in weight, this value is near to the theoretical value. Namely, 0.5 g of magnesium hydride and 30 g of a 10 wt% citric acid solution show quantity of generated hydrogen approaching the theoretical value. From this, in the case of using 15 g of citric acid solution to 1 g of magnesium hydride, for example, it is estimated that concentration of citric acid of approximately 40wt% is adequate. Alternatively, for example, using 10 g of the citric acid solution at a concentration of citric acid of 5wt%, it is estimated that quantity consumed of magnesium hydride of approximately 83 mg is adequate. If this method is employed as such, composition of the external medicine may be appropriately adjusted in light of quantity (concentration) of hydrogen to be generated and combination balance of each component. Note that with the external medicine according to the present invention, when an agent (first agent) containing a metal or metal hydride and an agent (second agent) containing a protic solvent (acid as needed) are mixed, it is preferable that for 1 g of the compound, approximately 0.0016 mg or more of hydrogen is generated, at least through calculation (quantity of hydrogen included in 1 g of saturated hydrogen water at 20 degrees Celsius and 1 atm). With less than this concentration, it is better to simply apply the saturated hydrogen water. Moreover, in light of usability and safety, quantity of generated hydrogen up to approximately 1000 mg is preferred. In other words, quantity of generated hydrogen from the compound resulting from mixing the first agent and the second agent is 0.0016 to 1000 mg/g, preferably 0.016 to 100 mg/g, more preferably 0.16 to 10 mg/g. Basically, while the quantity of generated hydrogen from the external medicine may be calculated from the metal or metal hydride, use of the aforementioned method while taking into consideration the quantity of the metal or metal hydride that generates desired hydrogen allows further proper adjustment of quantity of the metal or metal hydride, the concentration of acid, and quantity of solution of the acid. Note that at this time, the quantity of the metal or metal hydride should be adjusted so that hydrogen molecules generated theoretically from the first agent within the container including the first agent fall within the range of 1 ppm to 100% by volume, preferably 100 ppm to 80% by volume, more preferably 0.1 to 4% by volume, most preferably 1 to 4% by volume (see description in an embodiment of a simple hydrogen generator described later.)

An additional example of a representative embodiment is given forthwith.

According to the present invention, a simple hydrogen generator for treatment or prevention, which includes a container in which a metal such as magnesium or metal hydride as a first agent and a protic solvent (including acid as needed) such as water as a second agent is mixed together so as to generate active hydrogen or hydrogen molecules, may be structured. In other words, at the time of use, the first agent and the second agent are placed and mixed in a container so as to generate hydrogen, and a user intakes gas or vapors containing hydrogen via a hydrogen outlet on the top of the container or via a tube, mask, or the like installed at the outlet. Such generator is applicable as a device for the intake of gas or vapors such as a humidifier, gas cylinder, inhaler, nebulizer, or the like, and may be accordingly used to function as such a device. Note that considering handling safety, it is preferable that the metal or metal hydride within the first agent undergoes dust explosion prevention processing such as mixing of a dust explosion inhibitor therein, corrosion control processing, or dispersing within a liquid (particularly a liquid including an aprotic solvent or nonpolar solvent), or dispersing within an alkali liquid. Moreover, considering effect and handling safety, it is preferred that particle diameter and quantity consumed of the metal or metal hydride, or acid concentration, quantity consumed of solution, and the like are appropriately prepared such that concentration of hydrogen gas generated when the first agent and the second agent are mixed falls within the range of 1 ppm to 100% by volume, preferably 100 ppm to 80% by volume, more preferably 0.1 to 4% by volume, most preferably 1 to 4% by volume. The method described in [Measurement of quantity of generated hydrogen from metal magnesium and magnesium hydride using water replacement method] above may be applied to this preparation of component quantity consumed and concentration. For example, when 4% by volume of hydrogen is included in a 500 mL container, hydrogen generated due to reaction between the first agent and the second agent should be 20 mL (approximately 1.8 mg). Assuming that metal magnesium is used as the first agent, since quantity of hydrogen generated from 10 g of metal magnesium is calculatively 830 mg, as described in [Measurement of quantity of generated hydrogen from metal magnesium and magnesium hydride using water replacement method] above, approximately 21.7 mg of metal magnesium is theoretically necessary in order to ingenerate 1.8 mg of hydrogen. Moreover, similarly, as described in [Measurement of quantity of generated hydrogen from metal magnesium and magnesium hydride using water replacement method] above, assuming that a citric acid solution is used as the second agent, since 30 g of a 10 wt% citric acid solution is enough solution to completely dissolve 0.5 g of metal magnesium, calculated therefrom, it is estimated that approximately 1.3 g of a 10 wt% citric acid solution is enough solution to completely dissolve 21.7 g of metal magnesium. Furthermore, similarly, as described in [Measurement of quantity of generated hydrogen from metal magnesium and magnesium hydride using water replacement method] above, according to the knowledge here that hydrogen gas is generated in 2.5 minutes, a specification of the generator in which, for example, quantity consumed at one time should be the first agent including approximately 21.7 mg of metal magnesium and the second agent including approximately 1.3 g of the 10 wt% citric acid solution and hydrogen gas generated due to mixing of the first agent and the second agent should be inhaled over 2.5 minutes may be determined. Since generated hydrogen gas is activated through oxidation by free radicals and active oxygen species, such a simple hydrogen generator has applications such as, for example, inhibition of inflammation of the throat or lungs, improvement in anti-aging or maintaining beauty, inhibition of acute oxidant stress due to strenuous activity, ischemia-reperfusion injury, surgery, organ transplant, and the like, and inhibition of chronic oxidant stress due to drinking alcohol, smoking, sun bathing, and the like.

Note that 'external medicine' in this specification is not particularly limited as long as it is applicable to skin and mucous membranes, and includes makeup, pharmaceuticals, quasi drugs, and the like. Moreover, pharmaceutical forms thereof include arbitrary forms such as aqueous solution, solubilizer, emulsifier, oil, gel, paste, ointment, aerosol, vapor, water-oil two-layer type, water-oil-powder three-layer type, and the like. Furthermore, it may be used by impregnating gauze, a film, a sheet, a nonwoven fabric, or the like and attaching it to skin. The type of usage thereof is also arbitrary, and may be used in an arbitrary form such as skin toner, cosmetic oil, emulsion, facial cream, facial pack, serum, sun block, makeup base, foundation, massage agent, nail cream, toothpaste, mouth wash, patches, and air spray.

## Claims

1. An external medicine for treatment or prevention including an active hydrogen generating agent, wherein said external medicine makes contact with skin or a mucous membrane at any point during a process in which active hydrogen is generated.

2. The external medicine of Claim 1, wherein
the active hydrogen is hydrogen in a nascent state.

3. The external medicine of either Claim 1 or 2, wherein
the active hydrogen generating agent includes a catalyst for activating hydrogen molecules.

4. The external medicine of any one of Claims 1 to 3, wherein
the active hydrogen generating agent makes at least two substances touch each other so as to generate the active hydrogen.

5. The external medicine of any one of Claims 1 to 4, wherein
one of the two substances is a substance including at least one of an electron donor or a hydride ion donor, and the other of the two substances is a substance including a proton donor.

6. The external medicine of any one of Claims 1 to 5, wherein
the substance including at least one of an electron donor or a hydride ion donor is a substance including a metal.

7. The external medicine of any one of Claims 1 to 6, wherein
the substance including a metal is at least one of a metal hydride or a metal with a higher tendency of ionization than hydrogen.

8. The external medicine of any one of Claims 1 to 7, wherein
the metal is at least one of magnesium or magnesium hydride.

9. The external medicine of any one of Claims 1 to 8, wherein
at least either one of the electron donor or the hydride ion donor is retained in a medium including at least either one of an aprotic solvent or a nonpolar solvent.

10. The external medicine of any one of Claims 1 to 9, wherein
the proton donor is water.

11. The external medicine of any one of Claims 1 to 10, wherein
the water includes acid.

12. The external medicine of any one of Claims 1 to 11, wherein
the catalyst is a precious metal microparticle.

13. The external medicine of any one of Claims 1 to 12, wherein
the precious metal microparticle is any one of platinum, palladium, rhodium, iridium, ruthenium, gold, silver, rhenium, a salt thereof, an alloy compound, a complex compound, or a composite thereof.

14. The external medicine of any one of Claims 1 to 13, wherein
said external medicine is for treatment or prevention of atopic dermatitis.

15. The external medicine of any one of Claims 1 to 14, wherein
the external medicine is cosmetics.

16. The external medicine of any one of Claims 1 to 15, wherein
the active hydrogen generating agent includes liposome containing hydrogen.

17. An external medicine for treatment or prevention of gastric mucosa, including at least either a metal hydride or a metal with a higher tendency of ionization than hydrogen, and a catalyst or precious metal microparticle.

18. A simple hydrogen generator for treatment or prevention, comprising a metal or a metal hydride as a first agent, a protic solvent as a second agent, and a container for mixing the first agent and the second agent so as to generate active hydrogen or hydrogen molecules.
